# EUROPÄISCHE PATENTSCHRIFT

(11) **EP 0 882 129 B1**
(45) Veröffentlichungstag und Bekanntmachung des Hinweises auf die Patenterteilung: **18.01.2006**
(21) Anmeldenummer: 97904360.1
(22) Anmeldetag: 31.01.1997
(51) Int. Cl.: C12N 15/31, C12N 15/70, C12N 15/62, C07K 14/32, C12N 1/21, A61K 39/07

(54) **REKOMBINANTE EXPRESSION VON S-LAYER-PROTEINEN**
RECOMBINANT EXPRESSION OF S-LAYER PROTEINS
EXPRESSION PAR RECOMBINAISON DE PROTEINES DE COUCHE SUPERFICIELLE

(30) Priorität: 01.02.1996 DE 19603649
(43) Veröffentlichungstag der Anmeldung: 09.12.1998
(73) Patentinhaber: Lubitz, Werner, Dr., 1080 Wien (AT); NANO-S Biotechnologie GmbH, 1180 Wien (AT)
(72) Erfinder: LUBITZ, Werner, A-1080 Wien (AT); SLEYTR, Uwe, A-1170 Wien (AT); KUEN, Beatrix, A-1070 Wien (AT); TRUPPE, Michaela, A-4222 Luftenberg (AT); HOWORKA, Stefan, A-1130 Wien (AT); RESCH, Stepanka, A-1150 Wien (AT); SCHROLL, Gerhard, A-1070 Wien (AT); SARA, Margit, A-2230 Gänserndorf (AT)
(74) Vertreter: Weiss, Wolfgang
(86) Internationale Anmeldenummer: PCT/EP1997/000432
(87) Internationale Veröffentlichungsnummer: WO 1997/028263

(56) Entgegenhaltungen:
- WO-A-91/13155
- WO-A-95/19371
- DE-A- 4 425 527
- CANADIAN JOURNAL OF MICROBIOLOGY, Bd. 40, Nr. 9, September 1994, Seiten 777-782, XP000674689 BINGLE W. AND SMIT J.: "Alkaline phosphatase and a cellulase reporter protein are not exported from the cytoplasm when fused to large N-terminal portions of the Caulobacter crescentus surface (S)-layer protein" in der Anmeldung erwähnt
- MOLECULAR MICROBIOLOGY, Bd. 9, Nr. 1, 1993, Seiten 97-109, XP000674434 PEYRET J. ET AL.: "Characterization of the cspB gene encoding PS2, an ordered surface-layer protein in Corynebacterium glutamicum" in der Anmeldung erwähnt
- GENE, Bd. 145, Nr. 1, 1994, Seiten 115-120, XP002032765 KUEN B. ET AL.: "Sequence analysis of the sbsA gene encoding the 130-kDa surface-layer protein of bacillus stearothermophilus strain PV72" in der Anmeldung erwähnt
- JOURNAL OF BACTERIOLOGY, Bd. 176, Nr. 23, Dezember 1994, Seiten 7182-7189, XP000674692 SARA M. AND SLEYTR U.: "Comparative studies of S-layer proteins from Bacillus stearothermophilus strains expressed during growth in continuous culture under oxygen-limited and non-oxygen-limited conditions" in der Anmeldung erwähnt
- MOLECULAR MICROBIOLOGY, Bd. 19, Nr. 3, Februar 1996, Seiten 495-503, XP000675407 KUEN B. ET AL.: "Heterologous expression and self-assembly of the S-layer protein SbsA of Bacillus stearothermophilus in Escherichia coli"
- JOURNAL OF BACTERIOLOGY, Bd. 179, Nr. 5, März 1997, Seiten 1664-1670, XP000674432 KUEN B. ET AL.: "Molecular characterization of the Bacillus stearothermophilus PV72 S-layer gene sbsB induced by oxidative stress"
- TIBTECH, Bd. 15, Nr. 1, Januar 1997, Seiten 20-26, XP002032144 SLEYTR U. AND SARA M: "Bacterial and archaeal S-layer proteins: structure-function relationships and their biotechnological applications"

## Beschreibung

Die vorliegende Erfindung betrifft Verfahren zur rekombinanten Herstellung von S-Layer-Proteinen und modifizierten S-Layer-Proteinen in gram-negativen Wirtszellen, i.e. E. col Wirtszellen.

Kristalline bakterielle Zelloberflächenlayer (S-Layer) bilden in vielen Eubakterien und den allermeisten Archaebakterien die äußerste Zellwandkomponente (Sleytr et al. (1988), Crystalline Bacterial Cell Surface Layers, Springer Verlag Berlin; Messner und Sleytr, Adv.Mikrob.Physiol.33 (1992), 213-275). Die meisten der gegenwärtig bekannten S-Layer-Proteine sind aus identischen Proteinen bzw. Glykoproteinen zusammengesetzt, die scheinbare Molekulargewichte im Bereich von 40 000 bis 220 000 aufweisen. Die Komponenten von S-Layern sind selbst-assemblierend und die meisten Gitter haben eine schräge (p2), quadra-tische (p4) oder hexagonale (p6) Symmetrie. Die Funktionen von bakteriellen S-Layern sind immer noch nicht vollständig bekannt, aber aufgrund ihrer Lokalisierung an der Zelloberfläche dürften die porösen kristallinen S-Layer hauptsächlich als Schutzhüllen, Molekularsiebe oder zur Förderung der Zelladhä-sion und Oberflächenerkennung dienen.

Genetische Daten und Sequenzinformationen sind für verschiedene S-Layer-Gene aus Mikroorganismen bekannt. Eine Übersicht findet sich bei Peyret et al., Mol.Mikrobiol.9 (1993), 97-109. Auf diese Daten wird ausdrücklich Bezug genommen. Die Sequenz des für das S-Layer-Protein von B.stearothermophilus PV72 kodierenden Gens sbsA und ein Verfahren zu dessen Klonierung sind bei Kuen et al. (Gene 145 (1994), 115-120) angegeben.

B. stearothermophilus PV72 ist ein gram-positives Bakterium, das mit einem hexagonal angeordneten S-Layer bedeckt ist. Die Hauptkomponente des S-Layer ist ein 128 kd-Protein, bei dem es sich um das häufigste Protein in der Zelle mit einem Anteil von ungefähr 15% bezüglich der gesamten Proteinbestandteile handelt. Es sind verschiedene Stämme von B.stearothermophilus charakterisiert worden, die hinsichtlich des Typs von S-Layer-Gitter, dem Molekulargewicht und der Glykosilierung der S-Layer-Komponenten unterschiedlich sind (Messner und Sleytr (1992), supra).

Die deutsche Patentanmeldung P 44 25 527.6 offenbart den Signalpeptid-kodierenden Abschnitt des S-Layer-Gens aus B.stearothermophilus und die davon abgeleitete Aminosäuresequenz. Die Spaltstelle zwischen dem Signalpeptid und dem reifen Protein befindet sich zwischen Position 30 und 31 der Aminosäuresequenz. Die Signalpeptid-kodierende Nukleinsäure kann operativ mit einer Protein-kodierenden Nukleinsäure verknüpft werden und zur rekombinanten Herstellung von Proteinen in einem Verfahren verwendet werden, bei dem man eine transformierte Wirtszelle bereitstellt, die Wirtszelle unter Bedingungen kultiviert, die zu einer Expression der Nukleinsäure und zu einer Erzeugung und Sekretion des davon kodierten Polypeptids führen, und das resultierende Polypeptid aus dem Kulturmedium gewinnt. Als Wirtszellen werden vorzugsweise prokaryontische Organismen, insbesondere gram-positive Organismen der Gattung Bacillus genannt.

Überraschenderweise wurde festgestellt, daß die rekombinante Herstellung von S-Layer-Proteinen nicht nur in gram-positiven prokaryontischen Wirtszellen, sondern auch in gram-negativen prokaryontischen Wirtszellen möglich ist. Dabei bildet sich das S-Layer-Protein im Inneren der Wirtszelle nicht in Form von ungeordneten Einschlußkörpern, sondern unerwarteterweise in Form von geordneten monomolekularen Schichten.

Ein Gegenstand der vorliegenden Erfindung ist somit ein Verfahren zur rekombinanten Herstellung von S-Layer-Proteinen, dadurch gekennzeichnet, daß man (a) eine E. coli Wirtszelle bereitstellt, die transformiert ist mit
einer für ein S-Layer-Protein kodierenden Nukleinsäure, ausgewählt aus (i) einer Nukleinsäure, welche die von Position 1 bis 3684 in SEQ ID NO.1 gezeigte Nukleotidsequenz gegebenenfalls ohne den Signalpeptid-kodierenden Abschnitt umfasst, (b) die Wirtszelle unter solchen Bedingungen kultiviert, die zu einer Expression der Nukleinsäure und zu einer Erzeugung des davon kodierten Polypeptids führen und (c) das resultierende Polypeptid aus der Wirtszelle gewinnt.

Unter dem Begriff "stringente Hybridisierung" im Sinne der vorliegenden Erfindung versteht man, dass eine Hybridisierung auch nach dem Waschen bei 55 ° C, vorzugsweise 60 °C, in einem wässrigen Niedrigsalz-Puffer (z.B. 0,2 xSSC) noch auftritt (s. auch Sambrook et al. (1989), Molecular Cloning. A Labatory Manual).

Das erfindungsgemäße Verfahren wird in gram-negativen prokaryontischen Wirtszellen, i.e. E.coli Wirtszellen, durchgeführt. Dabei wird überraschenderweise im Zellinneren eine geordnete S-Layer-Proteinstruktur erhalten. Als Wirtszellen werden Enterobakterien, und zwar E.coli, verwendet. Besonders bevorzugt ist der E.coli-Stamm pop2135, der am 31.01.1996 bei der Deutschen Sammlung von Mikroorganismen und Zellkulturen GmbH, Maschero- der Weg 1b, D 38124 Braunschweig, unter dem Aktenzeichen DSM 10509 hinterlegt wurde.

Das erfindungsgemäße Verfahren kann auch zur Gewinnung rekombinanter S-Layer-Proteine eingesetzt werden. Hierzu verwendet man eine für das S-Layer-Protein kodierende Nukleinsäure, die eine oder mehrere Insertionen enthält, die für Peptid- oder Polypeptidsequenzen kodieren. Diese Insertionen können einerseits nur für Peptide mit wenigen Aminosäuren, z.B. 1-25 Aminosäuren, kodieren. Andererseits können die Insertionen auch für größere Polypeptide von z.B. bis zu 1000 Aminosäuren und vorzugsweise bis zu 500 Aminosäuren kodieren, ohne daß die Fähigkeit des S-Layer-Proteins zur Ausbildung einer korrekt gefalteten Struktur verlorengeht. Neben den Insertionen kann das rekombinante S-Layer-Protein auch Aminosäuresubstitutionen, insbesondere Substitutionen einzelner Aminosäuren im Bereich der Insertionsorte sowie gegebenenfalls Deletionen einzelner Aminosäuren oder kurzer Aminosäureabschnitte von bis zu 30 Aminosäuren aufweisen.

Als Insertionsstellen für Polypeptid-kodierende Sequenzen bevorzugt sind Bereiche zwischen den Positionen 1-1200 und 2200-3000 der in SEQ ID NO.1 gezeigten Nukleotidsequenz. Besonders bevorzugte Insertionsstellen sind die NruI-Schnittstelle an Position 582, die PvuII-Schnittstelle an Position 878, die SnaB-I-Schnittstelle an Position 917, die PvuII-Schnittstelle an Position 2504 und die PvulI-Schnittstelle an Position 2649. Die Insertion einer für Streptavidin kodierenden Nukleinsäuresequenz konnte bereits in die NruI-Schnittstelle an Position 581 gezeigt werden.

Die Peptid- oder Polypeptid-kodierenden Insertionen werden vorzugsweise ausgewählt aus Nukleotidsequenzen, die für Cysteinreste, Bereiche mit mehreren geladenen Aminosäuren, z.B. Arg, Lys, Asp oder Glu, oder Tyr-Resten, DNA-bindende Epitope, antigene, allergene oder immunogene Epitope, metallbindende Epitope, Streptavidin, Enzyme, Cytokine oder Antikörper-bindende Proteine kodieren.

Ein besonders bevorzugtes Beispiel für eine Insertion in die für das S-Layer-Protein kodierende Nukleinsäure ist eine für Streptavidin kodierende Nukleotidsequenz. Auf diese Weise können universelle Trägermoleküle erhalten werden, die zur Ankopplung von biotinylierten Reagenzien und zum Nachweis in immunologischen oder Hybridisierungstestverfahren geeignet sind.

Ein weiteres bevorzugtes Beispiel für Insertionen sind antigene allergene oder immunogene Epitope, z.B. Epitope aus pathogenen Mikroorganismen, wie etwa Bakterien, Pilzen, Parasiten etc. und Viren, oder Epitope aus Pflanzen oder Epitope gegen körpereigene Substanzen, z.B. Cytokine, sowie gegen Toxine, insbesondere Endotoxine. Besonders bevorzugte Beispiele für immunogene Epitope sind Epitope aus Herpesviren, wie etwa Herpesvirus 6 oder Pseudorabiesvirus (Lomniczi et al., J. Virol. 49 (1984), 970-979), insbesondere Epitope aus den Genen gB, gC oder/und gD, oder Maul- und Klauenseuchevirus (FMDV), insbesondere Epitope aus den Genabschnitten , die für VP1, VP2 oder/und VP3 kodieren. Die immunogenen Epitope können so ausgewählt werden, daß sie die Erzeugung einer Antikörpervermittelten Immunreaktion fördern oder/und die Erzeugung einer zellulären Immunreaktion, z.B. durch Stimulation von T-Zellen, fördern. Beispiele für geeignete allergene Epitope sind Birkenpollenallergene, z.B. Bet v I (Ebner et al., J. Immunol. 150 (1993) 1047-1054). Weiterhin besonders bevorzugt sind antigene Epitope, die in der Lage sind, aus Serum oder anderen Körperflüssigkeiten körpereigene oder körperfremde Substanzen wie etwa Cytokine oder Toxine zu binden und herauszufiltrieren. Derartige Epitope können Bestandteile von Cytokin- oder Toxinrezeptoren oder von Antikörpern gegen Cytokine oder Toxine umfassen.

Andererseits können die Insertionen auch für Enzyme kodieren. Bevorzugte Beispiele sind Enzyme zur Synthese von Polyhydroxybuttersäure, z.B. PHB-Synthase. Durch Einbau von PHB-Synthase in den S-Layer kann bei Zufuhr des Substrats Hydroxybuttersäure unter geeigneten Bedingungen eine molekulare Spinndüse entstehen. Ein weiteres bevorzugtes Beispiel für ein Enzym ist bakterielle Luciferase. Hier kann bei Zufuhr des Enzymsubstrates, eines Aldehyds, und in Anwesenheit von O₂ ein molekularer Laser erhalten werden.

Ebenfalls bevorzugt sind Insertionen, die für Cytokine, wie etwa Interleukine, Interferone oder Tumornekrosefaktoren kodieren. Diese Moleküle können beispielsweise in Kombination mit immunogenen Epitopen zur Herstellung von Vakzinen verwendet werden.

Schließlich sind auch Insertionen bevorzugt, die für Antikörper-bindende Proteine, wie etwa Protein-A oder Protein-G oder für DNA- oder/und metallbindende Epitope, wie etwa Leucin-Zipper, Zinkfinger etc. kodieren.

So wird durch die vorliegende Erfindung erstmals eine Zelle bereitgestellt, die im Cytoplasma immobilisierte rekombinante Polypeptide in nativer Form, z. B. aktive Enzyme enthält. Pro m² rekombinanten S-Layer können auf diese Weise 50.000 - 200.000, z. B. ca. 100.000 rekombinante Moleküle immobilisiert werden. Pro kg rekombinante E.coli Zellen können bis zu 3.000 m² S-Layer erhalten werden:

Vorzugsweise wird bei dem erfindungsgemäßen Verfahren die für das S-Layer-Protein kodierende Nukleinsäure in operativer Verknüpfung mit einer für ein Signalpeptid von gram-positiven Bakterien kodierenden Nukleinsäure verwendet, d.h. 5'-seitig von der S-Layer-Protein-kodierenden Nukleinsäure ist die Signalpeptid-kodierende Nukleinsäure angeordnet Überraschenderweise wurde nämlich festgestellt, daß die Anwesenheit derartiger Signalpeptidsequenzen, die in den erfindungsgemäß verwendeten gram-negativen Wirtszellen nicht abgespalten werden, die Stabilität der S-Layer-Strukturen verbessern kann. Besonders bevorzugt umfaßt die für das Signalpeptid kodierende Nukleinsäure (a) den Signalpeptid-kodierenden Abschnitt der in SEQ ID NO.1 dargestellten Nukleotidsequenz, (b) eine der Sequenz aus (a) im Rahmen der Degeneration des genetischen Codes entsprechende Nukleotidsequenz oder/und (c) eine zu den Sequenzen aus (a) oder/und (b) mindestens 80% und insbesondere mindestens 90% homologe Nukleotidsequenz.

Im erfindungsgemäßen Verfahren kann eine Nukleinsäure eingesetzt werden, die für ein rekombinantes S-Layer-Protein kodiert und ausgewählt ist aus (i) einer Nukleinsäure, welche die von Position 1 bis 3684 in SEQ ID NO.1 gezeigte Nukleotidsequenz gegebenenfalls ohne den Signalpeptid-kodierenden Abschnitt umfaßt.

In SEQ ID NO.1 ist die kodierende Nukleotidsequenz des S-Layer-Gens sbsA aus B. stearothermophilus einschließlich des -Signalpeptid-kodierenden Abschnitts gezeigt. Der Signalpeptid-kodierende Abschnitt reicht von Position 1-90 der in SEQ ID NO.1 gezeigten Nukleotidsequenz. Der für das reife SbsA-Polypeptid kodierende Abschnitt reicht von Position 91-3684.

Das sbsA-Gen von B.stearothermophilus kodiert für ein Protein mit insgesamt 1228 Aminosäuren einschließlich eines N-terminalen Signalpeptids mit 30 Aminosäuren (SEQ ID NO.2). Die Spaltstelle zwischen dem Signalpeptid und dem reifen Protein befindet sich zwischen Position 30 und 31 der Aminosäuresequenz. Das Signalpeptid weist eine basische aminoterminale Domäne, gefolgt von einer hydrophoben Domäne, auf.

Sequenzvergleiche mit anderen Signalpeptiden zeigen eine gewisse Homologie zu Signalpeptiden von extrazellulären Proteinen in Bazillen, wie etwa alkalische Phosphatase und neutrale Phosphatase von B. amyloliquefaciens (Vasantha et al., J.Bacteriol. 159 (1984), 811-819) sowie mit den Signalpeptiden für das B. sphaericus-Gen 125 (Bowditch et al., J. Bacteriol.171 (1989), 4178-4188) und das OWP-Gen von B.brevis (Tsuboi et al., J.Bacteriol.168 (1986), 365-373).

Im erfindungsgemäßen Verfahren kann auch ein rekombinanter Vektor eingesetzt werden, der mindestens eine Kopie der oben beschriebenen Nukleinsäure enthält. Der Vektor ist vorzugsweise in Prokaryonten replizierbar. Besonders bevorzugt ist der Vektor ein prokaryontisches Plasmid.

Dabei wird eine Wirtszelle erhalten, die mit einer Nukleinsäure oder einem rekombinanten Vektor, wie oben beschrieben, transformiert ist. Vorzugsweise ist die Zelle ein gram-negativer prokaryontischer Organismus und am meisten bevorzugt eine E.coli-Zelle. Die Zelle kann in ihrem Inneren eine rekombinante S-Layerstruktur enthalten. Verfahren zur Transformation von Zellen mit Nukleinsäuren sind allgemeiner Stand der Technik (siehe Sambrook et al., supra) und brauchen daher nicht erläutert zu werden.

Aus erfindungsgemäßen rekombinant hergestellten S-Layer-Proteinmolekülen kann eine rekombinante S-Layer-Struktur assembliert werden, die als Untereinheit mindestens ein erfindungsgemäß hergestelltes rekombinantes S-Layer-Protein enthält. Weiterhin ist bevorzugt, dass die S-layer-Struktur als "Verdünnungsmoleküle" auch nichtmodifizierte S-Layer-Proteine enthält. Die nichtmodifizierten S-Layer-Proteine liegen vorzugsweise in einem molaren Anteil von 10-99 % bezüglicher der.gesamten S-Layer-Proteine vor.

Die S-Layer-Struktur kann mehrere kovalent oder durch Affinitätsbindung miteinander verknüpfte Schichten umfassen. Kovalente Verknüpfungen können beispielsweise durch Insertionen von Cysteinresten und einer anschließenden Ausbildung von Cystinbrücken eingeführt werden. Verknüpfungen durch Affinitätsbindung umfassen beispielsweise Antikörper-Antigen-, Antikörper-Protein A- bzw. -Protein G- oder Streptavidin-Biotin-Wechselwirkungen.

S-Layer-Strukturen, die rekombinante S-Layer-Proteine enthalten, können gegebenenfalls auch in trägergebundener Form hergestellt werden. Hierzu kann die Reassemblierung der S-Layer-Struktur aus einzelnen Einheiten in Gegenwart eines Peptidoglycanträgers erfolgen, wobei beispielsweise Peptidoglycanschichten erzeugt werden, die auf einer oder auf beiden Seiten mit einer S-Layer-Struktur überzogen sind. Eine andere Möglichkeit zur Herstellung trägergebundener S-Layer-Strukturen besteht darin, eine S-Layer-Schicht an einer Grenzfläche zwischen zwei Medien, z.B. Wasser/Luft, zu erzeugen und diese Schicht auf einer Festphase, z.B. einer Filtermembran, zu immobilisieren (vgl. z.B. Pum und Sleytr (1994), Thin Solid Films 244, 882-886; Küpcü et al. (1995), Biochim. Biophys. Acta 1235, 263-269).

Die erfindungsgemäß hergestellten rekombinanten S-Layer-Proteine und S-Layer-Strukturen sind für eine Vielzahl von Anwendungen geeignet. Besonders bevorzugt ist die Verwendung als Vakzin oder Adjuvans, wobei man rekombinante S-Layer-Proteine verwendet, die immunogene Epitope von Pathogenen und/oder körpereigene immunstimulierende Polypeptide, wie etwa Cytokine, enthalten. Bei dieser Anwendung ist nicht unbedingt eine Reinigung der rekombinanten S-Layer-Proteine erforderlich. Stattdessen kann beispielsweise die Verwendung in Kombination mit einem Bakterienghost erfolgen, der ggf. in seiner Membran zusätzliche immunogene Epitope enthält.

Die Herstellung geeigneter "Bakterienghosts" ist beispielsweise in der internationalen Patentanmeldung PCT/EP91/00967 beschrieben, auf die hiermit Bezug genommen wird. Dort werden modifizierte Bakterien offenbart, erhältlich durch Transformation eines gram-negativen Bakteriums mit dem Gen eines lytisch wirkenden Membranproteins aus Bakteriophagen, mit dem Gen eines lytisch wirkenden Toxin-Freisetzungsproteins oder mit Genen, die Teilsequenzen davon, die für lytische Proteine kodieren, enthalten, Kultivierung des Bakteriums, Expression dieses Lyse-Gens und Isolierung des resultierenden Bakterienghosts aus dem Kulturmedium.

An die Membran dieser Bakterien kann, wie im europäischen Patent 0 516 655 beschrieben, ein rekombinantes Protein gebunden sein, das durch Expression einer rekombinanten DNA in diesen gram-negativen Bakterien erhältlich ist. Diese rekombinante DNA umfaßt eine erste DNA-Sequenz, welche für eine hydrophobe, nicht lytisch wirkende membranintegrierende Proteindomäne, die eine α-helikale Struktur besitzt und aus 14-20 Aminosäuren besteht, die N- und C-terminal von je 2-30 beliebigen Aminosäuren flankiert sein können, kodiert. Mit dieser ersten DNA-Sequenz in operativer Verknüpfung befindet sich eine zweite DNA-Sequenz, die für ein gewünschtes rekombinantes Protein kodiert. Weiterhin enthält das gram-negative Bakterium eine dritte DNA-Sequenz, die unter einer von den ersten und zweiten DNA-Sequezen getrennten Kontrolle steht und für ein lytisch wirkendes Membranprotein aus Bakteriophagen oder ein lytisch wirkendes Toxin-Freisetzungsprotein oder für deren lytisch wirkende Teile kodiert. Durch Expression und Lyse derartiger rekombinanter, gram-negativer Bakterien werden sog. "Bakterienghosts" erhalten, die eine intakte Oberflächenstruktur mit an die Oberfläche gebundenen immunogenen Epitopen enthalten.

Bei Kombination dieser Bakterienghosts mit erfindungsgemäß hergestellten rekombinanten S-Läyern können Vakzine und Adjuvantien erzeugt werden, die besonders vorteilhafte Eigenschaften aufweisen.

Eine weitere besonders bevorzugte Verwendung für rekombinante S-Layer-Proteine und S-Layer Strukturen ist die Verwendung als Enzymreaktor. Ein solcher Enzymreaktor kann beispielsweise von einer Zelle gebildet werden, die in ihrem Inneren eine erfindungsgemäße rekombinante S-Layer-Struktur enthält. Andererseits kann der Enzymreaktor auch aus isolierten und in vitro reassemblierten S-Layer-Strukturen oder Kombinationen verschiedender S-Layer-Strukturen gebildet werden.

Es wurde festgestellt, daß das gram-positive Bakterium B.stearothermophilus PV72 neben SbsA noch ein weiteres S-Layer-Protein enthält, das in der Folge als SbsB bezeichnet wird. (Sara und Sleytr (1994), J. Bacteriol. 176, 7182-7189). Durch Amplifikation unter Verwendung geeigneter Nukleinsäureprimer konnte das sbsB-Gen isoliert und charakterisiert werden. In SEQ ID NO.5 ist die kodierende Nukleotidsequenz des S-Läyer-Gens sbsB aus B.stearothermophilus einschließlich des Signalpeptid-kodierenden Abschnitts, der von Position 1-93 der Nukleinsäuresequenz reicht, gezeigt. In SEQ ID NO.6 ist die davon abgeleitete Aminosäuresequenz gezeigt. Das sbsB-Gen kodiert für ein Protein mit insgesamt 921 Aminosäuren einschließlich eines N-terminalen Signalpeptids mit 31 Aminosäuren.

Rekombinante S-Layer-Proteine sind erhältlich durch ein Verfahren, bei dem man
(a) eine Wirtszelle bereitstellt, die eine für ein S-Layer-Protein kodierende Nukleinsäure enthält, die innerhalb des für das S-Layer-Protein kodierenden Bereichs eine Peptid- oder Polypeptid-kodierende Insertion enthält,
(b) die Wirtszelle unter solchen Bedingungen kultiviert, die zu einer Expression der Nukleinsäure und zu einer Erzeugung des davon kodierten Polypeptids führen,und
(c) das resultierende Polypeptid aus der Wirtszelle oder dem Kulturmedium gewinnt.

In einer bevorzugten Ausführungsform dieses Verfahrens wird ein rekombinantes SbsA-S-Layer-Protein hergestellt, d.h. die für das rekombinante S-Layer-Protein kodierende Nukleinsäure wird ausgewählt aus
(i) einer Nukleinsäure, welche die von Position 1 bis 3684 in SEQ ID No.1 gezeigte Nukleotidsequenz gegebenenfalls ohne den Signalpeptid-kodierenden Abschnitt umfaßt.

Neben den rekombinanten SbsA und SbsB-S-Layer-Proteinen aus B.stearothermophilus können jedoch auch rekombinante S-Layer-Proteine aus anderen Organismen (vgl. z.B. Peyret et al., (1993), supra) hergestellt werden.

Die Herstellung der rekombinanten S-Layer-Proteine kann einerseits in einer heterologen Wirtszelle erfolgen, d.h. in einer Wirtszelle, die ursprünglich kein S-Layer-Gen enthält. Beispiele für solche heterologen Wirtszellen sind gram-negative prokaryontische Organismen, wie etwa E.coli.

Die heterologe Expression von S-Layer-Proteinen kann jedoch auch in gram-positiven prokaryontischen Organismen, wie etwa B. subtilis, erfolgen. Hierzu werden vorzugsweise Integrationsvektoren verwendet, die ein natives oder/und ein rekombinantes S-Layer-Gen enthalten. Bei Verwendung der nativen Signalsequenzen wird eine Sekretion der S-Layer-Proteine in den Kulturüberstand gebunden.

Oft ist jedoch die Herstellung der rekombinanten S-Layer-Proteine in homologen Wirtszellen bevorzugt, d.h. in Wirtszellen, die ursprünglich ein natürliches S-Layer-Gen enthalten. In einer Ausführungsform dieser homologen Expression wird das rekombinante S-Layer-Gen in die Wirtszelle so eingeführt, daß die Wirtszelle noch in der Lage ist, ein weiteres S-Layer-Gen zu exprimieren, das für ein nichtmodifiziertes S-Layer-Protein kodiert. Vorzugsweise ist das nichtmodifizierte S-Layer-Protein in der Lage, eine mit dem rekombinanten S-Layer-Protein kompatible S-Layer-Struktur auszubilden. Ein Beispiel für diese Ausführungsform der homologen Expression ist eine B.stearothermophilus PV72 Zelle, welche intakte natürliche sbsA- oder/und sbsB-Gene enthält, und die mit einem Plasmid transformiert ist, welches ein rekombinantes S-Layer-Gen enthält.

In einer zweiten Ausführungsform kann die homologe Expression in einer Wirtszelle erfolgen, in der das ursprünglich vorhandene intakte S-Layer-Gen inaktiviert wurde. Folglich wird in dieser Ausführungsform in der Wirtszelle kein weiteres S-Layer-Gen mehr exprimiert, das für ein nichtmodifiziertes S-Layer-Protein kodiert, welches in der Lage ist, eine mit dem rekombinanten S-Layer-Protein kompatible S-Layer-Struktur auszubilden. Ein spezifisches Beispiel für eine derartige Wirtszelle ist eine B.stearothermophilus PV72 Zelle, in deren Genom z.B. durch homologe Rekombination ein für ein rekombinantes S-Layer-Protein kodierendes Gen eingeführt wurde, welches das ursprüngliche S-Layer-Gen ersetzt. Ein weiterer Beispiel für eine derartige Wirtszelle ist eine B.stearothermophilus-Zelle, in der das native S-Layer-Gen z.B. durch ortsspezifische Mutagenese oder/und homologe Rekombination inaktiviert wurde und die mit einem ein rekombinantes S-Layer-Gen enthaltenden Vektor transformiert ist.

Bei der homologen Expression rekombinanter S-Layer-Gene werden als Wirtszellen üblicherweise gram-positive prokaryontische Organismen verwendet. Besonders bevorzugt als Wirtszelle ist B.stearothermophilus PV72, der bei hoher Temperatur in einem definierten synthetischen Medium (Schuster et al., (1995), Biotechnol, and Bioeng. 48: 66-77) kultiviert werden kann.

Weiterhin wird die vorliegende Erfindung durch die nachfolgenden Beispiele und Figuren erläutert. Es zeigen:
- SEQ ID NO.1: die vollständige Nukleotidsequenz des kodierenden Abschnitts des S-Layer-Gens sbsA von B.stearothermophilus;
- SEQ ID NO.2: die davon abgeleitete Aminosäuresequenz;
- SEQ ID NO.3: die Nukleotidsequenz des Primers T5-X;
- SEQ ID NO.4: die Nukleotidsequenz des Primers E;
- SEQ ID NO.5: die vollständige Nukleotidsequenz des kodierenden Abschnitts des S-Layer-Gens sbsB von B.stearothermophilus;
- SEQ ID NO.6: die davon abgeleitete Aminosäuresequenz;
- SEQ ID NO.7: die Nukleotidsequenz eines Teilfragments des Streptavidingens;
- SEQ ID NO.8: die Nukleotidsequenz des Primers NIS 2AG;
- SEQ ID NO.9: die Nukleotidsequenz des Primers LIS C3;
- Fig.1: eine schematische Darstellung des zur Herstellung des rekombinanten Vektors pBK4 verwendeten sbsA PCR-Fragments;
- Fig.2: eine schematische Darstellung von Peptidinsertionen in die Aminosäuresequenz des SbsA S-Layer-Proteins und
- Fig.3: eine schematische Darstellung von Aminosäuresubstitutionen und -insertionen in rekombinanten S-Layer-Proteinen.

### BEISPIELE:

### 1. Bakterienstämme, Medien und Plasmide

Gram-positive Bakterien des Stammes Bacillus stearothermophilus PV72 wurden bei 58°C in SVIII-Medium (Bartelmus und Perschak, Z.Zuckerind.7 (1957), 276-281) kultiviert. Bakterien des Stammes E.coli pop2135 (endA, thi, hsdR, malT, cI857, λpR, malPQ) wurden in LB-Medium kultiviert (Sambrook et al., (1989), supra). Zur Selektion von Transformanten wurde Ampicillin in einer Endkonzentration von 100 µg/ml dem Medium zugegeben. Das Plasmid pPLcAT10 (λpL, bla, colE1) (Stanssens et al., Gene 36 (1985), 211-223) wurde als Klonierungsvektor verwendet.

### 2. Manipulation von DNA-Fragmenten

Restriktionsanalyse von DNA, Agarosegelelektrophorese und Klonierung von DNA-Fragmenten wurden nach den bei Sambrook et al. (1989), supra, beschriebenen Standardmethoden durchgeführt.

Die Transformation von kompetenten Zellen erfolgte durch Elektroporation unter Verwendung eines Bio-Rad Genepulsers (Bio-Rad Laboratories, Richmond, Kalif., USA) nach Protokollen des Herstellers.

Plasmid-DNA wurde nach der Methode von Birnboim und Doly (Nucleic Acids Res.7 (1979), 1513-1523) isoliert. Chromosomale DNA wurde gemäß der bei Ausubel et al. (Current Protocols in Molecular Biology (1987), New York, John Wiley) beschriebenen Verfahren isoliert.

Restriktionsendonukleasen und andere Enzyme wurden von Boehringer Mannheim, New England Biolabs oder Stratagene bezogen und gemäß den Vorschriften der Hersteller eingesetzt.

### 3. DNA-Sequenzierung

Die DNA-Sequenzen der 5'- und 3'-Regionen (einschließlich des für die Signalsequenz kodierenden Bereichs) des Gens sbsA im Vektor pPLcAT10 wurde nach der Dideoxykettenterminationsmethode von Sanger et al. bestimmt. Die zur Sequenzierung verwendeten Primer wurden auf Basis der bereits publizierten sbsA-Sequenz (Kuen et al., Gene 145 (1994), 115-120) konstruiert.

### 4. PCR-Amplifikation von sbsA

Die PCR-Amplifikation des sbsA-Gens erfolgte in einem Reaktionsvolumen von 100 µl, in dem 200 µM Deoxynukleotide, 1U Pfu-Polymerase (Stratagene), 1x Pfu-Reaktionspuffer, jeweils 0.5µM Oligonukleotidprimer und 100 ng genomischer DNA aus B.stearothermophilus als Matrize vorhanden waren. Die Amplifikation wurde über 30 Zyklen in einem Thermocycler (Biomed Thermocycler 60) durchgeführt. Jeder Zyklus bestand aus einem Denaturierungsschritt von 1,5 min bei 95°C, einem Annealingschritt von 1 min bei 56°C und 1 min bei 50°C sowie einem Extensionsschritt von 2 min bei 72°C.

Als Primer wurden der im Sequenzprotokoll als SEQ ID NO.3 angegebene Primer T5-X, der den 5'-Bereich von sbsA flankiert und eine XbaI-Stelle enthält, sowie der im Sequenzprotokoll in SEQ ID NO.4 gezeigte Primer E verwendet, der die 20 Nukleotide stromabwärts gelegene Region des Transkriptionsterminators der sbsA-Sequenz flankiert und eine BamHI-Stelle enthält.

Die PCR-amplifizierten Produkte wurden auf einem 0.8% Agarosegel elektrophoretisch aufgetrennt und zur Klonierung unter Verwendung des Systems von Gene Clean (BIO101 La Jolla, Kalif., USA) zur Klonierung gereinigt.

### 5. Klonierung des sbsA-Gens in den Vektor pPLcAT10

Das durch PCR gewonnene sbsA-Gen mit einer Länge von 3,79 kb wurde gereinigt und mit den Restriktionsendonukleasen XbaI und BamHI gespalten. Das resultierende XbaI-BamHI-Fragment wurde in die entsprechenden Restriktionsstellen des Vektors pPLcAT10 kloniert, so daß das sbsA-Gen unter transkriptioneller Kontrolle des stromaufwärts gelegenen pL-Promotors war. Das ATG-Startkodon der sbsA-Sequenz wurde durch die Klonierungspozedur rekonstruiert. Die klonierte sbsA-Sequenz enthielt die N-terminale Signalsequenz von sbsA und endete 20 nt nach dem Transkriptionsterminator. Nach Ligation der Vektor-DNA mit dem sbsA-Fragment wurde der E.coli-Stamm pop2135 durch Elektrotransformation transformiert. Die resultierenden Klone wurden einer DNA-Restriktionsanalyse unterzogen. Ein positiver Klon wurde sequenziert, um die korrekten Sequenzübergänge an den 5'- und 3'-Enden zu verifizieren. Dieser Klon wurde als pBK4 bezeichnet.

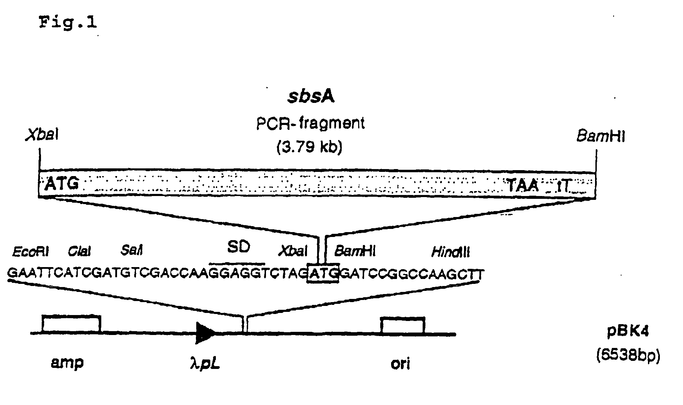

Eine schematische Darstellung des 3,79 kb XbaI sbsA-Fragments und seine Lokalisierung in der multiplen Klonierungsstelle des Plasmids pBK4 ist in Fig.1 dargestellt (Abkürzungen: tT: Transkriptionsterminator; ori: Ursprung der DNA-Replikation; amp: Ampicillinresistenzgen).

### 6. Rekombinante Expression des sbsA-Gens in E.coli

E.coli pop2135/pBK4-Zellen wurden bei 28°C bis zum Erreichen einer optischen Dichte OD₆₀₀ von 0,3 kultiviert. Dann wurde die Expression von sbsA durch Erhöhung der Kultivierungstemperatur von 28°C auf 42°C induziert. 1,5 ml Aliquots wurden vor bzw. 1, 2, 3 und 5 Stunden nach Induktion der sbsA-Expression entnommen. Als Kontrollen wurden E.coli pop2135/pPLcAT10 (kultiviert unter den gleichen Bedingungen) und B.stearothermophilus PV72 verwendet.

Kulturüberstände und Zellextrakte aus allen Proben wurden auf die Expression des S-Layer-Proteins durch SDS-PAGE und Western-Immunoblotting untersucht.

In Extrakten aus mit pBK4 transformierten E.coli-Zellen wurde eine zusätzliche starke Proteinbande mit dem gleichen Molekulargewicht wie das Wildtyp-SbsA-Protein gefunden. Es wurden keine Abbauprodukte von SbsA selbst in einem Zeitraum bis zu 5 Stunden nach der Induktion der Expression gefunden. Dies läßt vermuten, daß das S-Layer-Protein sbsA in E.coli stabil ist und nicht durch Proteasen abgebaut wird.

Es wurde eine densitometrische Bestimmung der relativen Menge an SbsA-Protein durchgeführt. Zu einem Zeitpunkt von 4 Stunden nach der Induktion lag das sbsA-Protein in einem Anteil von ca. 16% bezüglich des gesamten zellulären Proteins vor.

Das in E.coli erzeugte SbsA-Protein wanderte im SDS-Gel etwas langsamer als das natürliche SbsA-Protein aus B.stearothermophilus. Versuche zur Bestimmung der N-terminalen Aminosäuresequenz des SbsA-Proteins durch Edman-Abbau schlugen aufgrund einer Blockierung des N-Terminus fehl. Dies läßt vermuten, daß die Signalsequenz in E.coli nicht abgespalten wurde.

Auch eine Western Blot-Analyse von Gesamtzellextrakten und Kulturüberständen von E.coli/pBK4 ergab nur eine einzige sbsAspezifische Proteinbande mit einem etwas höheren Molekulargewicht als das Wildtyp-SbsA-Protein aus stearothermophilus.

Für den Western Blot wurden die Proteine auf eine Nitrozellulosemembran transferiert und mit einem polyklonalen Antiserum gegen SbsA aus Kaninchen inkubiert. Die Herstellung dieses Antiserums ist bei Egelseer et al. (J.Bacteriol.177 (1995), 1444-1451) beschrieben. Zum Nachweis gebundener SbsA-spezifischer Antikörper wurde ein Konjugat aus Ziegen-Anti-Kaninchen-IgG und alkalischer Phosphatase verwendet.

Aus Überständen von mit pBK4 transformierten E.coli-Zellen konnte auch nach Induktion der sbsA-Gen-Expression kein SbsA-Protein nachgewiesen werden. Daraus ist ersichtlich, daß SbsA nicht in das umgebende Medium exportiert wird.

### 7. Lokalisierung und Organisation des S-Layer-Proteins SbsA im Cytoplasma von E.coli

Zellen aus E.coli pop2135/pBK4, die aus Kulturen mit 1, 2, 3 und 5 Stunden nach Induktion der S-Layer-Proteinexpression geerntet wurden, wurden auf die intrazelluläre Organisation von sbsA untersucht. Nichtinduzierte, bei 28°C kultivierte Zellen und Zellen von B.stearothermophilus PV72 wurden als Kontrollen untersucht.

Hierzu wurden gesamte Zellen beider Organismen fixiert und in Spurrharz nach der Methode von Messner et al. (Int.J.Syst.Bacteriol.34 (1984), 202-210) fixiert und eingebettet. Anschließend wurden ultradünne Schnitte der eingebetteten Präparate hergestellt und mit Uranylacetat angefärbt.

Das Cytoplasma von nichtinduzierten E.coli-Zellen zeigte die typische granuläre Struktur, die sich auch bei einer Zunahme der OD der Suspensionen nicht änderte. Längsschnitte von E.coli-Zellen, die 1 Stunde nach Induktion der S-Layer-Proteinexpression geerntet wurden, zeigten parallele, blattartige Strukturen im Cytoplasma. Aus Querschnitten wurde ersichtlich, daß diese Strukturen eine konzentrische Anordnung zeigten.

Der Anteil blattartiger Strukturen zeigte einen deutlichen Anstieg zwischen 1 und 2 Stunden nach Induktion der sbsA-Expression und blieb danach im wesentlichen konstant.

Das in E.coli rekombinant hergestellte sbsA-Protein konnte auch durch Immunogoldmarkierung mit SbsA-spezifischen Antikörpern nachgewiesen werden. Auch mit dieser Nachweismethode wurde eine geordnete Struktur des rekombinant hergestellten SbsA-Proteins gefunden.

Aus diesen morphologischen Daten war klar ersichtlich, daß das SbsA-Protein sich nicht zu unregelmäßigen Einschlußkörpern aggregierte, sondern monomolekulare S-Layer-Kristalle bildete. Eine bemerkenswerte Eigenschaft der in E.coli assemblierten SbsA-S-Layer-Schichten war die konzentrische Anordnung in definierten Abständen. Das Vorhandensein der Signalsequenz störte die korrekte Assemblierung nicht.

### 8. Herstellung von rekombinanten sbsA-S-Layer-Genen

### 8.1 Insertion einer 6 bp langen DNA-Sequenz

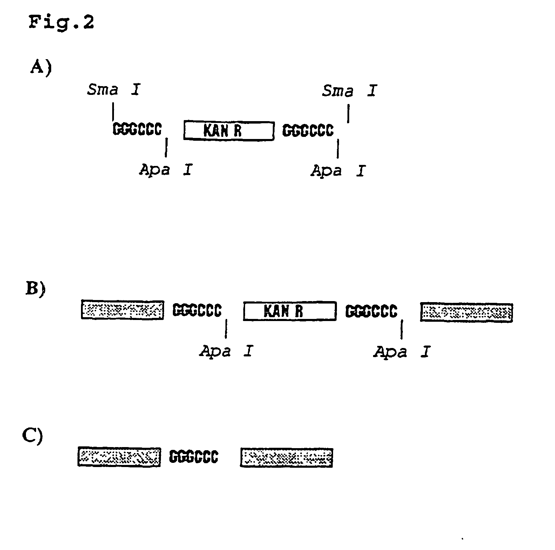

Für die ortsspezifische Insertionsmutagenese des sbsA-Gens wurde eine modifizierte Kanamycinkassette (1,3 kb) verwendet, die durch Spaltung des Plasmids pWJC3 (erhalten von W.T. McAllister, New York) durch SmaI isoliert wurde. Die Kassette wurde in fünf verschiedene glattendige Restriktionsstellen des sbsA-Gens ligiert, nämlich in die NruI-Stelle an Position bp 582 (pSL582), in die SnaBI-Stelle an Position bp 917 (pSL917) und in jede der PvuII-Stellen an Position bp 878 (pSL878), bp 2504 (pSL2504) und bp 2649 (pSL2649). Nach Selektion von Kanamycin-resistenten Klonen wurde die Kassette aus der Insertionsstelle durch Spaltung mit ApaI, gefolgt von einer Religation des S-Layer-Plasmids pBK4, entfernt. Durch die Herausschneide- und Religationsprozedur blieb eine Insertion von 6 bp CCCGGG (ApaI Restriktionsstelle) zurück. Das System dieser Linkerinsertion ist schematisch in Fig.2 dargestellt.

Die resultierenden rekombinanten S-Layer-Gene kodieren für modifizierte, um 2 Aminosäuren verlängerte sbsA-Proteine.

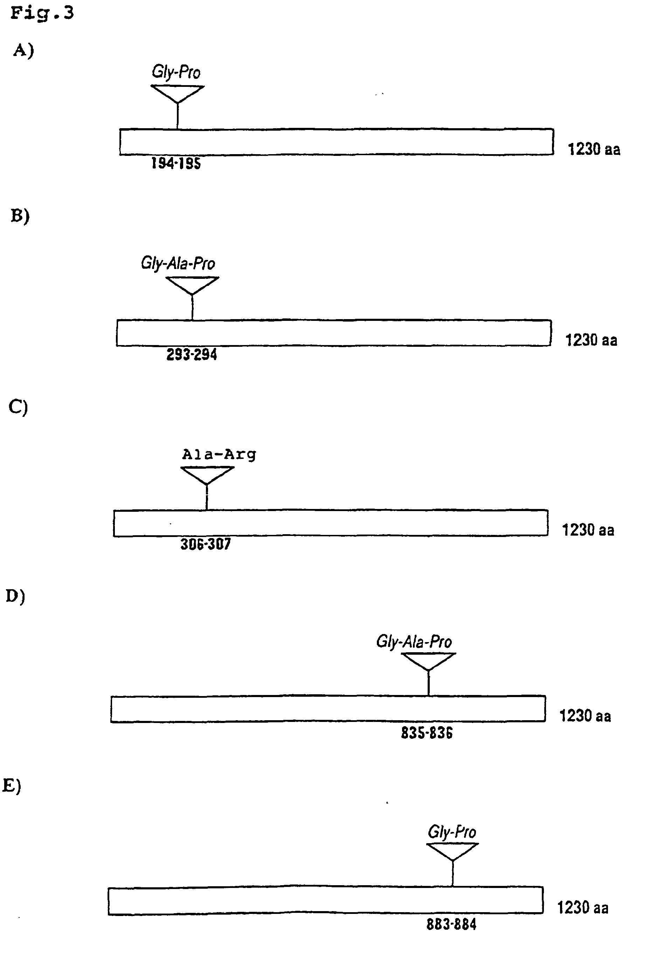

Die konkreten Änderungen in der Primärstruktur der sbsA-Proteine sind in Fig.3 gezeigt. Im Klon pSL582 führte die Insertion zur Einfügung von Glycin und Prolin zwischen den Aminosäuren 194 und 195 am N-Terminus des SbsA-Proteins. Die Aminosäuren Alanin und Arginin wurden im Klon pSL917 zwischen die Aminosäuren 306 und 307 eingefügt. Im Klon pSL2649 wurde eine Insertion von Glycin und Prolin zwischen die Aminosäuren an den Positionen 883 und 884 eingefügt. Eine Insertion von Alanin und Prolin zwischen den Aminosäuren 293 und 294 wurde im Klon pSL878 erhalten. Weiterhin wurde das Alanin an Position 293 durch Glycin ausgetauscht. Im Klon pSL2504 wurden die Aminosäuren Alanin und Prolin zwischen die Aminosäuren 835 und 836 eingeführt und das Alanin an Position 835 durch Glycin ersetzt.

Alle durch Insertionsmutagenese erhaltenen Klone behielten ihre Fähigkeit zur Synthese des S-Layer-Proteins.

Um die Fähigkeit der modifizierten Proteine zur Assemblierung in S-Layer-Strukturen nachzuweisen, wurden gemäß der unter Punkt 7. beschriebenen Prozedur ultradünne Längsschnitte von gesamten Zellen, die 4h unter induktiven Bedingungen kultiviert worden waren, hergestellt. Es wurde gefunden, daß das Cytoplasma aller 5 Klone mit parallelen, blattartigen Strukturen gefüllt ist, welche der Krümmung der Zellpole folgen.'Es gab keine morphologischen Unterschiede des Cytoplasma bei den 5 untersuchten verschiedenen Klonen. Es wurden genau die gleichen blattartigen Strukturen wie bei der Assemblierung des Wildtyp-SbsA-Protein in E.coli (Punkt 7.) festgestellt.

### 8.2 Insertion einer für Streptavidin kodierenden DNA-Sequenz

Um zu untersuchen, ob auch die Insertion größerer Proteinsequenzen in das SbsA-Protein toleriert werden kann, wurde ein mit ApaI-Linkern versehenes, für einen Teil von Streptavidin (160 Aminosäuren) kodierendes DNA-Fragment (SEQ ID No. 7) in die ApaI-Restriktionsstelle der in Beispiel Seite 1 hergestellten sbsA Klone psL582, pSL878, pSL917 und pSL2649 Gen inseriert. Die Insertion der Streptavidinsequenz erfolgte bei SL582 im Codon 197, bei pSL878 zwischen Codon 295 und 296, bei pSL917 zwischen Codon 308 und 309 und bei pSL2649 in Codon 886. Die Expression von SbsA-Streptavidin-Fusionsproteinen konnte bei allen Konstrukten durch SDS-Page und Immunoblots nachgewiesen werden. Durch EM-Analyse wurde festgestellt, daß eine Selbstassemblierung der S-Layerstruktur bei den Fusionsproteinen mit Insertionen im Codon 197 und zwischen den Codons 295 und 296 möglich war.

Die SbsA-Streptavidin-Fusionsproteine können als Monomere isoliert und zu homogenen SbsA-Streptavidin S-Layern oder zu gemischten SbsA-Streptavidin/SbsA-S-Layern reassembliert werden. Sie können zur Bindung biotinylierter Substanzen sowie zur Bestimmung der Bindekapazität von Enzymen und anderen gebundenen Molekülen eingesetzt werden.

### 8.3 Insertion einer für BetvI kodierenden DNA-Sequenz

Eine für den offenen Leserahmen von Betv1 (161 Aminosäuren), das Hauptpollenallergen der Birke, kodierende DNA Sequenz (Ferreira et al., J. Biol. Chem. 268 (1993), 19574-19580) wurde an der ApaI-Stelle in den sbsA-Klon pSL878 inseriert. Es konnte die Expression eines SbsA-Betv1-Fusionsproteins nachgewiesen werden, das eine immunologisch aktive Betv1-Domäne enthält.

Das resultierende Fusionsprotein kann für therapeutische oder diagnostische Zwecke eingesetzt werden. So kann durch Verabreichung des Fusionsproteins versucht werden, eine T_{H}2-gerichtete IgE-Antikörperreaktion in eine T_{H}1-vermittelte Reaktion gegen Betv1 umzuwandeln. Auf diese Weise kann das Auftreten der Symptome einer Pollenallergie unterdrückt werden. Weiterhin können SbsA-Betv1 Fusionsproteine zum Test von Anti-Betv1-Antikörperkonzentrationen oder/und zur Verringerung hoher Konzentrationen an Anti-Betv1-IgE verwendet werden.

### 8.4. Insertion für ein Pseudorabies Virus Antigen kodierender DNA-Sequenz

Es wurde eine Insertion der für das gB Epitop SmaBB (255 Aminosäuren) kodierenden DNA-Sequenz (Nucleotide 489-1224 entsprechend den Koordinaten gemäß EMBL-Seq: HEHSSGP2) von Pseudorabiesvirus in die SSpI-Stelle des sbsA-Gens nach nt 3484 (zwischen Codon 1161 und 1162) durchgeführt. Es konnte die Expression von SbsA-SmaBB Fusionsproteinen nachgewiesen werden.

Die Fusionsproteine können zum Testen gB-spezifischer Immunreaktionen verwendet werden. Eine Westernblot Analyse mit einem monoklonalen Antikörper, welcher der inserierten Sequenz entspricht, zeigte die immunologische Aktivität der viralen Domäne innerhalb der rekombinanten SbsA-SmaBB Proteine.

### 8.5 Insertion einer für PHB-Synthase (PhbC) von Alcaligenes eutrophus H16 kodierenden DNA-Sequenz

Eine regelmäßige Anordnung von Polypeptidstrukturen mit enzymatischer Aktivität an der Oberfläche von S-Layern ist ein wichtiges Ziel zur Herstellung immobilisierter Enzyme innerhalb einer lebenden Zelle und im Falle der 590 Aminosäuren langen PHB Synthase zur Herstellung einer molekularen Maschine für eine Biopolymersynthese.

Das phbC Gen wurde durch PCR aus dem Plasmid p4A (Janes et al., Molecular characterisation of the poly-β-hydroxy-butyrate biosynthesis in Alcaligenes eutrophus H16. In: Novel Biodegradable Microbial Polymers (HRSG. Daves, E. A.), pp 175-190 (1990), Kluver, Dordrecht) als 1770 nt langes DNA-Fragment (entsprechend einem offenen Leserahmen von 590 Aminosäuren) isoliert und in die ApaI-Schnittstelle des sbsA-Klons pSL878 inseriert, wobei das Plasmid pSbsA-PhbC erhalten wurde. In einer mit diesem Plasmid transformierten E.coli Zelle konnte die Expression eines SbsA-PhbC-Fusionsproteins-mit ca. 195 kD nachgewiesen werden. Bei Insertion von 2 Kopien des phbC-Gens hintereinander in die ApaI-Stelle von pSL878 konnte die Expression eines Fusionsproteins mit ca. 260 kD nachgewiesen werden.

Für einen funktionellen Test der enzymatischen Aktivität des SbsA-PhbC Konstrukts wurden die E.coli Zellen, die das Plasmid pSbsA-PhbC enthielten, mit dem Plasmid pUMS cotransformiert, welches die β-Ketothiolase (PhbA) und die Acetoacetyl-CoA-Reduktase (PhbB) von A. eutrophus (Kalousek et al., Genetic engineering of PHB-synthase from Alcaligenes eutrophus H16. In: Proceedings of the International Symposium on Bacterial Polyhydroxy-alkanoates, pp 426-427 (1993), HRSG. Schlegel H. G., Steinbüchel A. Goltze Druck, Göttingen) enthält. Die Poly-β-hydroxybutyrat-Bildung in den cotransformierten E.coli Zellen konnte durch Anfärbung mit Sudanschwarz, Gaschromatographie und Elektronenmikroskopie nachgewiesen werden. Diese Befunde zeigen, daß das SbsA-PhbC-Konstrukt enzymatisch aktiv ist und erfolgreiches Beispiel zur Immobilisierung von Enzymen auf intrazellulären S-Layer-Matrizen darstellt.

### 8.6 Insertion einer für ein bakterielles Luciferasegen codierenden DNA-Sequenz

Ein monocistronisches LuxAB Gen mit einer Länge von 2.070 nt, welches ein aus den beiden Untereinheiten LuxB und LuxB der bakteriellen Luciferase aus Vibrio harveyi bestehendes Fusionsprotein LuxAB enthält, wurde aus dem Plasmid pT7-mut3 (Boylan et al., J. Biol. Chem. 264 (1989), 1915-1918) durch PCR isoliert und in die ApaI Stelle des in Beispiel 8.1 hergestellten Klons pSL878 inseriert, wobei das Plasmid pBK878-LuxAB erhalten wurde. In einer mit diesem Plasmid transformierten E.coli Zelle konnte die Expression eines SbsA-PhbC Fusionsproteins mit ca. 207 kD nachgewiesen werden. Die enzymatische Aktivität des Fusionsproteins wurde durch die bei Boylan et al., Supra, beschriebene Methode gezeigt.

### 9. Isolierung und Charakterisierung des sbsB-Gens

Als Grundlage für die Isolierung des sbsB-Gens diente die Aminosäuresequenz des N-Terminus sowie die Sequenz von drei internen Peptiden des SbsB-Proteins. Von diesen Peptidsequenzen ausgehend wurden degenerierte Oligonukleotidprimer konstruiert und für die PCR eingesetzt. Auf diese Weise wurde ein 1076 bp langes PCR-Fragment aus der chromosomalen DNA von B. stearothermophilus amplifiziert, kloniert und sequenziert (entsprechend Position 100-1176 der in SEQ ID NO.5 gezeigten Sequenz).

Für die Amplifikation der 5'- und 3'-seitigen Abschnitte des sbsB-Gens wurde die Methode der inversen PCR angewendet und mit Hilfe verschiedener Primerkombinationen schrittweise überlappende DNA-Fragmente erhalten und sequenziert.

Zur Amplifizierung des vollständigen sbsB-Gens wurden als Primer der im Sequenzprotokoll als SEQ ID NO.8 angegebene Primer NIS 2AG, der den 5'-Bereich von sbsB enthält, sowie der im Sequenzprotokoll in SEQ ID NO.9 angegebene Primer LIS C3 verwendet, der den 3'-Bereich von sbsB enthält.

Das auf diese Weise erhaltene PCR-Fragment, welches die in SEQ ID NO.5 gezeigte Nukleotidsequenz mit 5'- und 3'-BamHI-Restriktionsschnittstellen enthält, wurde wie im Beispiel 5 beschrieben in den Vektor pPLcAT10 kloniert, in dem die Expression unter Kontrolle des Lambda PL-Promotors erfolgte.

Weiterhin wurde das sbsB-PCR-Fragment mit 5' seitiger EcoRI- und 3'seitiger BamHI-Schnittstelle in den Vektor pUC18 kloniert, in dem die Expression unter Kontrolle des lac-Promotors erfolgte.

Der Nachweis der sbsB-Expression erfolgte wie in den Beispielen 6 und 7 beschrieben durch SDS-Gelelektrophorese und Elektronenmikroskopie.

### 10. Herstellung von rekombinanten sbsB-S-Layer-Genen

Analog der in Beispiel 8 beschriebenen Methoden wurden rekombinante sbsB Gene hergestellt.

So wurde gemäß der in Beispiel 8.1 beschriebenen Methode eine 6 nt lange, eine ApaI-Restriktionsschnittstelle enthaltende DNA-Sequenz an verschiedenen Positionen in das sbsB-Layer-Gen eingeführt. Auf diese Weise wurden die rekombinanten sbsB Klone pAK407, pAK481 und pAK1582 mit ApaI-Schnittstellen bei nt 407 (Codon 136), 481 (Codon 161/162) und 1582 (Codon 528/529) erhalten. Diese durch Insertionsmutagene erhaltenen Klone behielten ihre Fähigkeit zur Synthese des S-Layerproteins und zur Ausbildung von S-Layerstrukturen.

Analog der in Beispiel 8.2 beschriebenen Methode wurde ein für Streptavidin kodierendes DNA-Fragment in die ApaI-Restriktionsstellen der sbsB-Klone pAK407 bzw. pAK481 eingeführt.

Analog Beispiel 8.4 wurde eine für das gB-Epitop SmaBB codierende DNA-Sequenz in die ApaI-Schnittstellen der sbsB Klone pAK481 und pAK1582 eingeführt. In den mit den resultierenden rekombinanten Plasmiden transformierten E.coli Zellen konnte die Expression von sbsB-SmaB Fusionsproteinen mit ca. 130 kD nachgewiesen werden. Bei Insertion von zwei Kopien des SmaBB Epitops hintereinander in die ApaI-Schnittstelle von pAK481 konnte die Expression eines Fusionproteins mit ca. 157 kD nachgewiesen werden. Die SmaBB Domänen der Fusionsproteine wurden von spezifischen Antikörpern erkannt.

Analog zu Beispiel 8.6 konnte bei Insertion der LuxAB Sequenz in die ApaI-Schnittstelle von pAK407 die Expression eines 175 kD SbsB-LuxAB Fusionproteins nachgewiesen werden.

### 11. Hereologe Expression von sbsA und sbsB in Bacillus subtilis

Zur heterologen Expression von sbsA und sbsB in B. subtilis wurde der Integrationsvektor pX (Kim, L., Mogk, A. und Schumann W., Gene 181 (1996), 71-76: A xylose-inducible Bacillus subtilis integration vector and its application) verwendet. In den resultierenden rekombinanten Expressionsvektoren befinden sich die S-Layer Gene unter der transkriptionellen Kontrolle des xyl Promotors. Transformanten von B. subtilis mit einem im Chromosom integrierten S-Layergen zeigten eine durch Zugabe von Xylose zum Wachstumsmedium induzierbare Expression von großen Mengen an S-Layerproteinen im Überstand der Zellen. Dies zeigt, daß die Signalsequenzen von sbsA und sbsB von der B. subtilis Zelle erkannt werden.

Auf analoge Weise konnte eine heterologe Expression rekombinanter sbsA und sbsB Layergene in B. subtilis erreicht werden.

## Patentansprüche

1. Verfahren zur Herstellung von S-Layer-Proteinen,
**dadurch gekennzeichnet,**
**dass** man
(a) eine E. coli Wirtszelle bereitstellt, die transformiert ist mit einer für ein S-Layer-Protein kodierenden Nukleinsäure, ausgewählt aus
(i) einer Nukleinsäure, welche die von Position 1 bis Position 3684 in SEQ ID NO. 1 gezeigte Nukleotidsequenz gegebenenfalls ohne den Signalpeptid-kodierenden Abschnitt umfasst,
(b) die Wirtszelle unter solchen Bedingungen kultiviert, die zu einer Expression der Nukleinsäure und zu einer Erzeugung des davon kodierten Polypeptids führen und
(c) das resultierende Polypeptid aus der Wirtszelle gewinnt.

2. Verfahren nach Anspruch 1,
**dadurch gekennzeichnet,**
**dass** man das Polypeptid in Form einer assemblierten S-Layer-Struktur aus dem Inneren der Wirtszelle gewinnt,

3. Verfahren nach einem der Ansprüche 1 bis 2,
**dadurch gekennzeichnet,**
**dass** die für das S-Layer-Protein kodierende Nukleinsäure eine oder mehrere Insertionen enthält, die für Peptid- oder Polypeptidsequenzen kodieren.

4. Verfahren nach Anspruch 3,
**dadurch gekennzeichnet,**
**dass** die Insertionen ausgewählt werden aus Nukleotidsequenzen, die für Cysteinreste, Bereiche mit mehreren geladenen Aminosäuren oder Tyr-Resten, DNA-bindende Epitope, metallbindende Epitope, immunogene Epitope, allergene Epitope, antigene Epitope, Streptavidin, Enzyme, Cytokine oder Antikörper-bindende Proteine kodieren.

5. Verfahren nach Anspruch 4,
**dadurch gekennzeichnet,**
**dass** die Insertionen für Polypeptide kodieren, ausgewählt aus Streptavidin, immunogenen Epitopen aus Herpesviren, insbesondere Herpesvirus 6 oder FMDV, Enzymen, wie etwa Polyhydroxybuttersäuresynthase oder bakterielle Luciferase, Cytokinen, wie etwa Interleukine, Interferone oder Tumornekrosefaktoren, Antikörperbindenden Proteinen, wie etwa Protein A oder Protein G, antigenen Epitopen, die an Cytokine oder Endotoxine binden, und metallbindenden Epitopen.

6. Verfahren nach einem der Ansprüche 1 bis 5,
**dadurch gekennzeichnet,**
**dass** 5'-seitig der für das S-Layer-Protein kodierenden Nukleinsäure in operativer Verknüpfung eine für ein gram-positives Signalpeptid kodierende Nukleinsäure angeordnet ist.

7. Verfahren nach Anspruch 6,
**dadurch gekennzeichnet,**
**dass** die für das Signalpeptid kodierende Nukleinsäure
(a) den Signalpeptid-kodierenden Abschnitt der in SEQ ID NO.1 dargestellten Nukleotidsequenz,
(b) eine der Sequenz aus (a) im Rahmen der Degeneration des genetischen Codes entsprechende Nukleotidsequenz oder/und
(c) eine zu den Sequenzen aus (a) oder/und (b) mindestens 80% homologe Nukleotidsequenz umfasst.

## Claims

1. Process for the production of S-layer proteins
**characterized in that**
(a) an E. coli host cell is provided which is transformed with a nucleic acid coding for an S-layer protein which is selected from
(i) a nucleic acid which comprises the nucleotide sequence from position 1 to 3684 shown in SEQ ID NO.1 optionally without the signal peptide-coding section,
(b) the host cell is cultured under conditions which lead to an expression of the nucleic acid and to production of the polypeptide coded by it and
(c) the resulting polypeptide is isolated from the host cell.

2. Process as claimed in claim 1,
**characterized in that**
the polypeptide is isolated from the interior of the host cell in the form of an assembled S-layer structure.

3. Process as claimed in one of the claims 1 to 2,
**characterized in that**
the nucleic acid coding for the S-layer protein contains one or several insertions which code for peptide or polypeptide sequences.

4. Process as claimed in claim 3,
**characterized in that**
the insertions are selected from nucleotide sequences which code for cysteine residues, regions with several charged amino acids or Tyr residues, DNA-binding epitopes, metal-binding epitopes, immunogenic epitopes, allergenic epitopes, antigenic epitopes, streptavidin, enzymes, cytokines or antibody-binding proteins.

5. Process as claimed in claim 4,
**characterized in that**
the insertions code for polypeptides selected from streptavidin, immunogenic epitopes from herpes viruses, in particular herpes virus 6 or FMDV, enzymes such as polyhydroxybutyric acid synthase or bacterial luciferase, cytokines such as interleukins, interferons or tumour necrosis factors, antibody-binding proteins such as protein A or protein G, antigenic epitopes which bind to cytokines or endotoxins, and metal-binding epitopes.

6. Process as claimed in one of the claims 1 to 5,
**characterized in that**
a nucleic acid coding for a gram-positive signal peptide is arranged in operative linkage on the 5' side of the nucleic acid coding for the S-layer protein.

7. Process as claimed in claim 6,
**characterized in that**
the nucleic acid coding for the signal peptide comprises
(a) the signal peptide-coding section of the nucleotide sequence shown in SEQ ID NO.1.
(b) a nucleotide sequence corresponding to the sequence from (a) within the degeneracy of the genetic code or/and
(c) a nucleotide sequence that is at least 80 % homologous to the sequences from (a) or/and (b).

## Revendications

1. Procédé de préparation de protéines S-Layer , **caractérisé en ce que**
(a) on prépare une cellule hôte d'*E*. *coli*, qui est transformée avec un acide nucléique codant pour une protéine S-Layer, choisi parmi
(i) un acide nucléique qui comprend la séquence de nucléotides indiquée de la position 1 à la position 3684 dans SEQ ID NO. 1 éventuellement sans le fragment codant pour le peptide signal,
(b) on cultive la cellule hôte dans des conditions qui entraînent une expression de l'acide nucléique et une production du polypeptide codé par celui-ci, et
(c) on obtient le polypeptide résultant à partir de la cellule hôte.

2. Procédé selon la revendication 1, **caractérisé en ce que** l'on obtient le polypeptide sous forme d'une structure S-Layer assemblée à partir de l'intérieur de la cellule hôte.

3. Procédé selon l'une des revendications 1 à 2, **caractérisé en ce que** l'acide nucléique codant pour la protéine S-Layer contient un ou plusieurs inserts qui codent pour des séquences peptidiques ou polypeptidiques.

4. Procédé selon la revendication 3, **caractérisé en ce que** les inserts sont choisis parmi des séquences de nucléotides qui codent pour des résidus de cystéine, des domaines ayant plusieurs aminoacides chargés ou des résidus Tyr, des épitopes se liant à l'ADN, des épitopes se liant à des métaux, des épitopes immunogènes, des épitopes allergènes, des épitopes antigéniques, la streptavidine, des enzymes, des cytokines ou des protéines se liant aux anticorps.

5. Procédé selon la revendication 4, **caractérisé en ce que** les inserts codent pour des polypeptides choisis parmi la streptavidine, des épitopes immunogènes d'herpèsvirus, en particulier de l'herpèsvirus 6 ou du FMDV, des enzymes comme, par exemple, une acide polyhydroxybutyrique-synthase ou une luciférase bactérienne, des cytokines comme, par exemple, des interleukines, des interférons ou des facteurs de nécrose tumorale, des protéines se liant aux anticorps comme, par exemple, la protéine A ou la protéine G, des épitopes antigéniques qui se lient à des cytokines ou à des endotoxines, et des épitopes se liant à des métaux.

6. Procédé selon l'une des revendications 1 à 5, **caractérisé en ce qu'**un acide nucléique codant pour un peptide signal gram-positif est lié de façon opérationnelle en 5' de l'acide nucléique codant pour la protéine S-Layer.

7. Procédé selon la revendication 6, **caractérisé en ce que** l'acide nucléique codant pour le peptide signal comprend
(a) le fragment codant pour le peptide signal de la séquence de nucléotides représentée par SEQ ID NO. 1,
(b) une séquence de nucléotides correspondant à la séquence de (a) dans le cadre de la dégénérescence du code génétique et/ou
(c) une séquence de nucléotides homologue à au moins 80 % aux séquences de (a) et/ou (b).


### SEQUENZPROTOKOLL

(1) ALLGEMEINE ANGABEN:
   (i) ANMELDER:
      (A) NAME: Werner Lubitz
      (B) STRASSE: Schoenborngasse 12/7
      (C) ORT: Wien
      (E) LAND: Austria
      (F) POSTLEITZAHL: 1080

      (A) NAME: Uwe Sleytr
      (B) STRASSE: Parhamerplatz 10
      (C) ORT: Wien
      (E) LAND: Austria
      (F) POSTLEITZAHL: 1170
   (ii) BEZEICHNUNG DER ERFINDUNG: Rekombinante Expression von S-Layer-Proteinen
   (iii) ANZAHL DER SEQUENZEN: 9
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 3687 Basenpaare
      (B) ART: Nucleotid
      (C) STRANGFORM: beides
      (D) TOPOLOGIE: linear
   (vi) URSPRÜNLICHE HERKUNFT:
      (A) ORGANISMUS: Bacillus stearothermophilus
      (B) STAMM: PV72
   (vii) UNMITTELBARE HERKUNFT:
      (B) CLON (E) : sbsA
   (ix) MERKMAL:
      (A) NAME/SCHLÜSSEL: CDS
      (B) LAGE:1..3684
   (ix) MERKMAL:
      (A) NAME/SCHLÜSSEL: sig peptide
      (B) LAGE:1..90
   (ix) MERKMAL:
      (A) NAME/SCHLÜSSEL: mat_peptide

      (B) LAGE:91..3684
   (xi) SEQUENZBESCHREIBUNG: SEQ ID NO: 1:
   ATG GAT AGG AAA AAA GCT GTG AAA CTA GCA ACA GCA AGT GCT ATT GCA      48
   Met Asp Arg Lys Lys Ala Val Lys Leu Ala Thr Ala Ser Ala Ile Ala
   -30                 -25                 -20                 -15
   GCA AGT GCA TTT GTC GCT GCA AAT CCA AAC GCT TCT GAA GCG GCT ACA      96
   Ala Ser Ala Phe Val Ala Ala Asn Pro Asn Ala Ser Glu Ala Ala Thr
                   -10                 -5                   1
   GAT GTA GCA ACA GTA GTA AGC CAA GCA AAA GCA CAG TTC AAA AAA GCA     144
   Asp Val Ala Thr Val Val Ser Gln Ala Lys Ala Gln Phe Lys Lys Ala
               5                   10                  15
   TAC TAT ACT TAC AGC CAT ACA GTA ACG GAA ACT GGT GAA TTC CCA AAC     192
   Tyr Tyr Thr Tyr Ser His Thr Val Thr Glu Thr Gly Glu Phe Pro Asn
       20                  25                  30
   ATT AAC GAT GTA TAT GCT GAA TAC AAC AAA GCG AAA AAA CGA TAC CGT     240
   Ile Asn Asp Val Tyr Ala Glu Tyr Asn Lys Ala Lys Lys Arg Tyr Arg
   35                  40                  45                  50
   GAT GCG GTA GCA TTA GTG AAT AAA GCA GGT GGC GCG AAA AAA GAC GCT     288
   Asp Ala Val Ala Leu Val Asn Lys Ala Gly Gly Ala Lys Lys Asp Ala
                   55                  60                  65
   TAC TTA GCT GAT TTA CAA AAA GAA TAT GAA ACT TAC GTT TTC AAA GCA     336
   Tyr Leu Ala Asp Leu Gln Lys Glu Tyr Glu Thr Tyr Val Phe Lys Ala
               70                  75                  80
   AAC CCT AAA TCT GGC GAA GCT CGT GTA GCA ACT TAC ATC GAT GCT TAC     384
   Asn Pro Lys Ser Gly Glu Ala Arg Val Ala Thr Tyr Ile Asp Ala Tyr
           85                  90                  95
   AAC TAT GCA ACA AAA TTA GAC GAA ATG CGC CAA GAG CTA GAG GCT GCT     432
   Asn Tyr Ala Thr Lys Leu Asp Glu Met Arg Gln Glu Leu Glu Ala Ala
       100                 105                 110
   GTT CAA GCA AAA GAT TTA GAA AAA GCA GAA CAA TAC TAT CAC AAA ATT     480
   Val Gln Ala Lys Asp Leu Glu Lys Ala Glu Gln Tyr Tyr His Lys Ile
   115                 120                 125                 130
   CCT TAT GAA ATT AAA ACT CGC ACA GTC ATT TTA GAT CGC GTA TAT GGT     528
   Pro Tyr Glu Ile Lys Thr Arg Thr Val Ile Leu Asp Arg Val Tyr Gly
                   135                 140                 145
   AAA ACA ACT CGT GAT TTA CTT CGC TCT ACA TTT AAA GCA AAA GCA CAA     576
   Lys Thr Thr Arg Asp Leu Leu Arg Ser Thr Phe Lys Ala Lys Ala Gln
               150                 155                 160
   GAA CTT CGC GAC AGC TTA ATT TAT GAT ATT ACC GTT GCA ATG AAA GCG     624
   Glu Leu Arg Asp Ser Leu Ile Tyr Asp Ile Thr Val Ala Met Lys Ala
           165                 170                 175
   CGC GAA GTA CAA GAC GCT GTG AAA GCA GGC AAT TTA GAC AAA GCT AAA      672
   Arg Glu Val Gln Asp Ala Val Lys Ala Gly Asn Leu Asp Lys Ala Lys
       180                 185                 190
   GCT GCT GTT GAT CAA ATC AAT CAA TAC TTA CCA AAA GTA ACA GAT GCT      720
   Ala Ala Val Asp Gln Ile Asn Gln Tyr Leu Pro Lys Val Thr Asp Ala
   195                 200                 205                 210
   TTC AAA ACT GAA CTA ACA GAA GTA GCG AAA AAA GCA TTA GAT GCA GAT      768
   Phe Lys Thr Glu Leu Thr Glu Val Ala Lys Lys Ala Leu Asp Ala Asp
                   215                 220                 225
   GAA GCT GCG CTT ACT CCA AAA GTT GAA AGT GTA AGT GCG ATT AAC ACT      816
   Glu Ala Ala Leu Thr Pro Lys Val Glu Ser Val Ser Ala Ile Asn Thr
               230                 235                 240
   CAA AAC AAA GCT GTT GAA TTA ACA GCA GTA CCA GTG AAC GGA ACA CTA      864
   Gln Asn Lys Ala Val Glu Leu Thr Ala Val Pro Val Asn Gly Thr Leu
           245                 250                 255
   AAA TTA CAA CTT TCA GCT GCT GCA AAT GAA GAT ACA GTA AAC GTA AAT      912
   Lys Leu Gln Leu Ser Ala Ala Ala Asn Glu Asp Thr Val Asn Val Asn
       260                 265                 270
   ACT GTA CGT ATC TAT AAA GTG GAC GGT AAC ATT CCA TTT GCC CTT AAT      960
   Thr Val Arg Ile Tyr Lys Val Asp Gly Asn Ile Pro Phe Ala Leu Asn
   275                 280                 285                 290
   ACG GCA GAT GTT TCT TTA TCT ACA GAC GGA AAA ACT ATC ACT GTG GAT     1008
   Thr Ala Asp Val Ser Leu Ser Thr Asp Gly Lys Thr Ile Thr Val Asp
                   295                 300                 305
   GCT TCA ACT CCA TTC GAA AAT AAT ACG GAG TAT AAA GTA GTA GTT AAA     1056
   Ala Ser Thr Pro Phe Glu Asn Asn Thr Glu Tyr Lys Val Val Val Lys
               310                 315                 320
   GGT ATT AAA GAC AAA AAT GGC AAA GAA TTT AAA GAA GAT GCA TTC ACT     1104
   Gly Ile Lys Asp Lys Asn Gly Lys Glu Phe Lys Glu Asp Ala Phe Thr
           325                 330                 335
   TTC AAG CTT CGA AAT GAT GCT GTA GTT ACT CAA GTG TTT GGA ACT AAT     1152
   Phe Lys Leu Arg Asn Asp Ala Val Val Thr Gln Val Phe Gly Thr Asn
       340                 345                 350
   GTA ACA AAC AAC ACT TCT GTA AAC TTA GCA GCA GGT ACT TTC GAC ACT     1200
   Val Thr Asn Asn Thr Ser Val Asn Leu Ala Ala Gly Thr Phe Asp Thr
   355                 360                 365                 370
   GAC GAT ACT TTA ACA GTA GTA TTT GAT AAG TTG TTA GCA CCT GAA ACT     1248
   Asp Asp Thr Leu Thr Val Val Phe Asp Lys Leu Leu Ala Pro Glu Thr
                   375                 380                 385
   GTA AAC AGC TCG AAC GTT ACT ATT ACA GAT GTT GAA ACT GGA AAA CGC     1296
   Val Asn Ser Ser Asn Val Thr Ile Thr Asp Val Glu Thr Gly Lys Arg
               390                 395                 400
   ATT CCA GTA ATT GCA TCT ACT TCT GGT TCT ACA ATT ACT ATT ACG TTA     1344
   Ile Pro Val Ile Ala Ser Thr Ser Gly Ser Thr Ile Thr Ile Thr Leu
           405                 410                 415
   AAA GAA GCG TTA GTA ACT GGT AAA CAA TAT AAA CTT GCT ATC AAT AAT     1392
   Lys Glu Ala Leu Val Thr Gly Lys Gln Tyr Lys Leu Ala Ile Asn Asn
       420                 425                 430
   GTT AAA ACA TTA ACT GGT TAC AAT GCA GAA GCT TAC GAG TTA GTG TTC     1440
   Val Lys Thr Leu Thr Gly Tyr Asn Ala Glu Ala Tyr Glu Leu Val Phe
   435                 440                 445                 450
   ACT GCA AAC GCA TCA GCA CCA ACT GTT GCT ACC GCT CCT ACT ACT TTA 1488
   Thr Ala Asn Ala Ser Ala Pro Thr Val Ala Thr Ala Pro Thr Thr Leu
   455 460 465
   GGT GGT ACA ACT TTA TCT ACT GGT TCT CTT ACA ACA AAT GTT TGG GGT 1536
   Gly Gly Thr Thr Leu Ser Thr Gly Ser Leu Thr Thr Asn Val Trp Gly
   470 475 480
   AAA TTG GCT GGT GGT GTG AAT GAA GCT GGA ACT TAT TAT CCT GGT CTT 1584
   Lys Leu Ala Gly Gly Val Asn Glu Ala Gly Thr Tyr Tyr Pro Gly Leu
   485 490 495
   CAA TTC ACA ACA ACG TTT GCT ACT AAG TTA GAC GAA TCT ACT TTA GCT 1632
   Gln Phe Thr Thr Thr Phe Ala Thr Lys Leu Asp Glu Ser Thr Leu Ala
   500 505 510
   GAT AAC TTT GTA TTA GTT GAA AAA GAA TCT GGT ACA GTT GTT GCT TCT 1680
   Asp Asn Phe Val Leu Val Glu Lys Glu Ser Gly Thr Val Val Ala Ser
   515 520 525 530
   GAA CTA AAA TAT AAT GCA GAC GCT AAA ATG GTA ACT TTA GTG CCA AAA 1728
   Glu Leu Lys Tyr Asn Ala Asp Ala Lys Met Val Thr Leu Val Pro Lys
   535 540 545
   GCG GAC CTT AAA GAA AAT ACA ATC TAT CAA ATC AAA ATT AAA AAA GGC 1776
   Ala Asp Leu Lys Glu Asn Thr Ile Tyr Gln Ile Lys Ile Lys Lys Gly
   550 555 560
   TTG AAG TCC GAT AAA GGT ATT GAA TTA GGC ACT GTT AAC GAG AAA ACA 1824
   Leu Lys Ser Asp Lys Gly Ile Glu Leu Gly Thr Val Asn Glu Lys Thr
   565 570 575
   TAT GAG TTC AAA ACT CAA GAC TTA ACT GCT CCT ACA GTT ATT AGC GTA 1872
   Tyr Glu Phe Lys Thr Gln Asp Leu Thr Ala Pro Thr Val Ile Ser Val
   580 585 590
   ACG TCT AAA AAT GGC GAC GCT GGA TTA AAA GTA ACT GAA GCT CAA GAA 1920
   Thr Ser Lys Asn Gly Asp Ala Gly Leu Lys Val Thr Glu Ala Gln Glu
   595 600 605 610
   TTT ACT GTG AAG TTC TCA GAG AAT TTA AAT ACA TTT AAT GCT ACA ACC 1968
   Phe Thr Val Lys Phe Ser Glu Asn Leu Asn Thr Phe Asn Ala Thr Thr
   615 620 625
   GTT TCG GGT AGC ACA ATC ACA TAC GGT CAA GTT GCT GTA GTA AAA GCG 2016
   Val Ser Gly Ser Thr Ile Thr Tyr Gly Gln Val Ala Val Val Lys Ala
   630 635 640
   GGT GCA AAC TTA TCT GCT CTT ACA GCA AGT GAC ATC ATT CCA GCT AGT 2064
   Gly Ala Asn Leu Ser Ala Leu Thr Ala Ser Asp Ile Ile Pro Ala Ser
   645 650 655
   GTT GAA GCG GTT ACT GGT CAA GAT GGA ACA TAC AAA GTG AAA GTT GCT 2112
   Val Glu Ala Val Thr Gly Gln Asp Gly Thr Tyr Lys Val Lys Val Ala
   660 665 670
   GCT AAC CAA TTA GAA CGT AAC CAA GGG TAC AAA TTA GTA GTG TTC GGT 2160
   Ala Asn Gln Leu Glu Arg Asn Gln Gly Tyr Lys Leu Val Val Phe Gly
   675 680 685 690
   AAA GGT GCA ACA GCT CCT GTT AAA GAT GCT GCA AAT GCA AAT ACT TTA 2208
   Lys Gly Ala Thr Ala Pro Val Lys Asp Ala Ala Asn Ala Asn Thr Leu
   695 700 705
   GCA ACT AAC TAT ATC TAT ACA TTT ACA ACT GAA GGT CAA GAC GTA ACA 2256
   Ala Thr Asn Tyr Ile Tyr Thr Phe Thr Thr Glu Gly Gln Asp Val Thr
   710 715 720
   GCA CCA ACG GTT ACA AAA GTA TTC AAA GGT GAT TCT TTA AAA GAC GCT      2304
   Ala Pro Thr Val Thr Lys Val Phe Lys Gly Asp Ser Leu Lys Asp Ala
           725                 730                 735
   GAT GCA GTT ACT ACA CTT ACG AAC GTT GAT GCA GGT CAA AAA TTC ACT      2352
   Asp Ala Val Thr Thr Leu Thr Asn Val Asp Ala Gly Gln Lys Phe Thr
       740                 745                 750
   ATC CAA TTT AGC GAA GAA TTA AAA ACT TCT AGT GGT TCT TTA GTG GGT      2400
   Ile Gln Phe Ser Glu Glu Leu Lys Thr Ser Ser Gly Ser Leu Val Gly
   755                 760                 765                 770
   GGC AAA GTA ACT GTC GAG AAA TTA ACA AAC AAC GGA TGG GTA GAT GCT      2448
   Gly Lys Val Thr Val Glu Lys Leu Thr Asn Asn Gly Trp Val Asp Ala
                   775                 780                 785
   GGT ACT GGA ACA ACT GTA TCA GTT GCT CCT AAG ACA GAT GCA AAT GGT      2496
   Gly Thr Gly Thr Thr Val Ser Val Ala Pro Lys Thr Asp Ala Asn Gly
               790                 795                 800
   AAA GTA ACA GCT GCT GTG GTT ACA TTA ACT GGT CTT GAC AAT AAC GAC      2544
   Lys Val Thr Ala Ala Val Val Thr Leu Thr Gly Leu Asp Asn Asn Asp
           805                 810                 815
   AAA GAT GCG AAA TTG CGT CTG GTA GTA GAT AAG TCT TCT ACT GAT GGA      2592
   Lys Asp Ala Lys Leu Arg Leu Val Val Asp Lys Ser Ser Thr Asp Gly
       820                 825                 830
   ATT GCT GAT GTA GCT GGT AAT GTA ATT AAG GAA AAA GAT ATT TTA ATT      2640
   Ile Ala Asp Val Ala Gly Asn Val Ile Lys Glu Lys Asp Ile Leu Ile
   835                 840                 845                 850
   CGT TAC AAC AGC TGG AGA CAC ACT GTA GCT TCT GTG AAA GCT GCT GCT      2688
   Arg Tyr Asn Ser Trp Arg His Thr Val Ala Ser Val Lys Ala Ala Ala
                   855                 860                 865
   GAC AAA GAT GGT CAA AAC GCT TCT GCT GCA TTC CCA ACA AGC ACT GCA      2736
   Asp Lys Asp Gly Gln Asn Ala Ser Ala Ala Phe Pro Thr Ser Thr Ala
               870                 875                 880
   ATT GAT ACA ACT AAG AGC TTA TTA GTT GAA TTC AAT GAA ACT GAT TTA      2784
   Ile Asp Thr Thr Lys Ser Leu Leu Val Glu Phe Asn Glu Thr Asp Leu
           885                 890                 895
   GCG GAA GTT AAA CCT GAG AAC ATC GTT GTT AAA GAT GCA GCA GGT AAT      2832
   Ala Glu Val Lys Pro Glu Asn Ile Val Val Lys Asp Ala Ala Gly Asn
       900                 905                 910
   GCG GTA GCT GGT ACT GTA ACA GCA TTA GAC GGT TCT ACA AAT AAA TTT      2880
   Ala Val Ala Gly Thr Val Thr Ala Leu Asp Gly Ser Thr Asn Lys Phe
   915                 920                 925                 930
   GTA TTC ACT CCA TCT CAA GAA TTA AAA GCT GGT ACA GTT TAC TCT GTA      2928
   Val Phe Thr Pro Ser Gln Glu Leu Lys Ala Gly Thr Val Tyr Ser Val
                   935                 940                 945
   ACA ATT GAC GGT GTG AGA GAT AAA GTA GGT AAC ACA ATC TCT AAA TAC      2976
   Thr Ile Asp Gly Val Arg Asp Lys Val Gly Asn Thr Ile Ser Lys Tyr
               950                 955                 960
   ATT ACT TCG TTC AAG ACT GTA TCT GCG AAT CCA ACG TTA TCT TCA ATC      3024
   Ile Thr Ser Phe Lys Thr Val Ser Ala Asn Pro Thr Leu Ser Ser Ile
           965                 970                 975
   AGC ATT GCT GAC GGT GCA GTT AAC GTT GAC CGT TCT AAA ACA ATT ACA      3072
   Ser Ile Ala Asp Gly Ala Val Asn Val Asp Arg Ser Lys Thr Ile Thr
       980                 985                 990
   ATT GAA TTC AGC GAT TCA GTT CCA AAC CCA ACA ATC ACT CTT AAG AAG      3120
   Ile Glu Phe Ser Asp Ser Val Pro Asn Pro Thr Ile Thr Leu Lys Lys
   995                 1000                1005                1010
   GCT GAC GGA ACT TCA TTT ACT AAT TAC ACT TTA GTA AAT GTA AAT AAT      3168
   Ala Asp Gly Thr Ser Phe Thr Asn Tyr Thr Leu Val Asn Val Asn Asn
                   1015                1020                1025
   GAA AAT AAA ACA TAC AAA ATT GTA TTC CAC AAA GGT GTA ACA CTT GAC      3216
   Glu Asn Lys Thr Tyr Lys Ile Val Phe His Lys Gly Val Thr Leu Asp
               1030                1035                1040
   GAG TTT ACT CAA TAT GAG TTA GCA GTT TCA AAA GAT TTT CAA ACT GGT      3264
   Glu Phe Thr Gln Tyr Glu Leu Ala Val Ser Lys Asp Phe Gln Thr Gly
           1045                1050                1055
   ACT GAT ATT GAT AGC AAA GTT ACA TTC ATC ACA GGT TCT GTT GCT ACT      3312
   Thr Asp Ile Asp Ser Lys Val Thr Phe Ile Thr Gly Ser Val Ala Thr
       1060                1065                1070
   GAC GAA GTA AAA CCT GCT CTA GTA GGC GTT GGT TCA TGG AAT GGA ACA      3360
   Asp Glu Val Lys Pro Ala Leu Val Gly Val Gly Ser Trp Asn Gly Thr
   1075                1080                1085                1090
   AGC TAT ACT CAG GAT GCT GCA GCA ACA CGA CTT CGG TCT GTA GCT GAC      3408
   Ser Tyr Thr Gln Asp Ala Ala Ala Thr Arg Leu Arg Ser Val Ala Asp
                   1095                1100                1105
   TTC GTT GCG GAG CCA GTT GCC CTT CAA TTC TCA GAA GGT ATC GAT TTA      3456
   Phe Val Ala Glu Pro Val Ala Leu Gln Phe Ser Glu Gly Ile Asp Leu
               1110                1115                1120
   ACG AAT GCA ACT GTG ACA GTA ACA AAT ATT ACT GAT GAT AAA ACT GTT      3504
   Thr Asn Ala Thr Val Thr Val Thr Asn Ile Thr Asp Asp Lys Thr Val
           1125                1130                1135
   GAA GTT ATT TCA AAA GAG AGT GTA GAC GCA GAC CAT GAT GCA GGT GCT      3552
   Glu Val Ile Ser Lys Glu Ser Val Asp Ala Asp His Asp Ala Gly Ala
       1140                1145                1150
   ACT AAG GAG ACA TTA GTA ATT AAC ACA GTT ACT CCT TTA GTA CTT GAT      3600
   Thr Lys Glu Thr Leu Val Ile Asn Thr Val Thr Pro Leu Val Leu Asp
   1155                1160                1165                1170
   AAC AGC AAG ACT TAT AAG ATT GTT GTA AGT GGA GTT AAA GAT GCA GCA      3648
   Asn Ser Lys Thr Tyr Lys Ile Val Val Ser Gly Val Lys Asp Ala Ala
                   1175                1180                1185
   GGT AAT GTT GCA GAT ACT ATT ACA TTC TAT ATT AAG TAA                  3687
   Gly Asn Val Ala Asp Thr Ile Thr Phe Tyr Ile Lys
               1190                1195
(2) ANGABEN ZU SEQ ID NO: 2:
   (i) SEQUENZKENNZEICHEN:
      (A) LÄNGE: 1228 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Asp Arg Lys Lys Ala Val Lys Leu Ala Thr Ala Ser Ala Ile Ala
   -30                 -25                 -20                 -15
   Ala Ser Ala Phe Val Ala Ala Asn Pro Asn Ala Ser Glu Ala Ala Thr
                   -10                 -5                   1
   Asp Val Ala Thr Val Val Ser Gln Ala Lys Ala Gln Phe Lys Lys Ala
            5                  10                  15
   Tyr Tyr Thr Tyr Ser His Thr Val Thr Glu Thr Gly Glu Phe Pro Asn
        20                  25                  30
   Ile Asn Asp Val Tyr Ala Glu Tyr Asn Lys Ala Lys Lys Arg Tyr Arg
    35                  40                  45                  50
   Asp Ala Val Ala Leu Val Asn Lys Ala Gly Gly Ala Lys Lys Asp Ala
                    55                  60                  65
   Tyr Leu Ala Asp Leu Gln Lys Glu Tyr Glu Thr Tyr Val Phe Lys Ala
                70                  75                  80
   Asn Pro Lys Ser Gly Glu Ala Arg Val Ala Thr Tyr Ile Asp Ala Tyr
            85                  90                  95
   Asn Tyr Ala Thr Lys Leu Asp Glu Met Arg Gln Glu Leu Glu Ala Ala
       100                 105                 110
   Val Gln Ala Lys Asp Leu Glu Lys Ala Glu Gln Tyr Tyr His Lys Ile
   115                 120                 125                 130
   Pro Tyr Glu Ile Lys Thr Arg Thr Val Ile Leu Asp Arg Val Tyr Gly
                   135                 140                 145
   Lys Thr Thr Arg Asp Leu Leu Arg Ser Thr Phe Lys Ala Lys Ala Gln
               150                 155                 160
   Glu Leu Arg Asp Ser Leu Ile Tyr Asp Ile Thr Val Ala Met Lys Ala
           165                 170                 175
   Arg Glu Val Gln Asp Ala Val Lys Ala Gly Asn Leu Asp Lys Ala Lys
       180                 185                 190
   Ala Ala Val Asp Gln Ile Asn Gln Tyr Leu Pro Lys Val Thr Asp Ala
   195                 200                 205                 210
   Phe Lys Thr Glu Leu Thr Glu Val Ala Lys Lys Ala Leu Asp Ala Asp
                   215                 220                 225
   Glu Ala Ala Leu Thr Pro Lys Val Glu Ser Val Ser Ala Ile Asn Thr
               230                 235                 240
   Gln Asn Lys Ala Val Glu Leu Thr Ala Val Pro Val Asn Gly Thr Leu
           245                 250                 255
   Lys Leu Gln Leu Ser Ala Ala Ala Asn Glu Asp Thr Val Asn Val Asn
       260                 265                 270
   Thr Val Arg Ile Tyr Lys Val Asp Gly Asn Ile Pro Phe Ala Leu Asn
   275                 280                 285                 290
   Thr Ala Asp Val Ser Leu Ser Thr Asp Gly Lys Thr Ile Thr Val Asp
                   295                 300                 305
   Ala Ser Thr Pro Phe Glu Asn Asn Thr Glu Tyr Lys Val Val Val Lys
               310                 315                 320
   Gly Ile Lys Asp Lys Asn Gly Lys Glu Phe Lys Glu Asp Ala Phe Thr
   325 330 335
   Phe Lys Leu Arg Asn Asp Ala Val Val Thr Gln Val Phe Gly Thr Asn
   340 345 350
   Val Thr Asn Asn Thr Ser Val Asn Leu Ala Ala Gly Thr Phe Asp Thr
   355 360 365 370
   Asp Asp Thr Leu Thr Val Val Phe Asp Lys Leu Leu Ala Pro Glu Thr
   375 380 385
   Val Asn Ser Ser Asn Val Thr Ile Thr Asp Val Glu Thr Gly Lys Arg
   390 395 400
   Ile Pro Val Ile Ala Ser Thr Ser Gly Ser Thr Ile Thr Ile Thr Leu
   405 410 415
   Lys Glu Ala Leu Val Thr Gly Lys Gln Tyr Lys Leu Ala Ile Asn Asn
   420 425 430
   Val Lys Thr Leu Thr Gly Tyr Asn Ala Glu Ala Tyr Glu Leu Val Phe
   435 440 445 450
   Thr Ala Asn Ala Ser Ala Pro Thr Val Ala Thr Ala Pro Thr Thr Leu
   455 460 465
   Gly Gly Thr Thr Leu Ser Thr Gly Ser Leu Thr Thr Asn Val Trp Gly
   470 475 480
   Lys Leu Ala Gly Gly Val Asn Glu Ala Gly Thr Tyr Tyr Pro Gly Leu
   485 490 495
   Gln Phe Thr Thr Thr Phe Ala Thr Lys Leu Asp Glu Ser Thr Leu Ala
   500 505 510
   Asp Asn Phe Val Leu Val Glu Lys Glu Ser Gly Thr Val Val Ala Ser
   515 520 525 530
   Glu Leu Lys Tyr Asn Ala Asp Ala Lys Met Val Thr Leu Val Pro Lys
   535 540 545
   Ala Asp Leu Lys Glu Asn Thr Ile Tyr Gln Ile Lys Ile Lys Lys Gly
   550 555 560
   Leu Lys Ser Asp Lys Gly Ile Glu Leu Gly Thr Val Asn Glu Lys Thr
   565 570 575
   Tyr Glu Phe Lys Thr Gln Asp Leu Thr Ala Pro Thr Val Ile Ser Val
   580 585 590
   Thr Ser Lys Asn Gly Asp Ala Gly Leu Lys Val Thr Glu Ala Gln Glu
   595 600 605 610
   Phe Thr Val Lys Phe Ser Glu Asn Leu Asn Thr Phe Asn Ala Thr Thr
   615 620 625
   Val Ser Gly Ser Thr Ile Thr Tyr Gly Gln Val Ala Val Val Lys Ala
   630 635 640
   Gly Ala Asn Leu Ser Ala Leu Thr Ala Ser Asp Ile Ile Pro Ala Ser
   645 650 655
   Val Glu Ala Val Thr Gly Gln Asp Gly Thr Tyr Lys Val Lys Val Ala
   660 665 670
   Ala Asn Gln Leu Glu Arg Asn Gln Gly Tyr Lys Leu Val Val Phe Gly
   675 680 685 690
   Lys Gly Ala Thr Ala Pro Val Lys Asp Ala Ala Asn Ala Asn Thr Leu
   695 700 705
   Ala Thr Asn Tyr Ile Tyr Thr Phe Thr Thr Glu Gly Gln Asp Val Thr
   710 715 720
   Ala Pro Thr Val Thr Lys Val Phe Lys Gly Asp Ser Leu Lys Asp Ala
   725 730 735
   Asp Ala Val Thr Thr Leu Thr Asn Val Asp Ala Gly Gln Lys Phe Thr
   740 745 750
   Ile Gln Phe Ser Glu Glu Leu Lys Thr Ser Ser Gly Ser Leu Val Gly
   755 760 765 770
   Gly Lys Val Thr Val Glu Lys Leu Thr Asn Asn Gly Trp Val Asp Ala
   775 780 785
   Gly Thr Gly Thr Thr Val Ser Val Ala Pro Lys Thr Asp Ala Asn Gly
   790 795 800
   Lys Val Thr Ala Ala Val Val Thr Leu Thr Gly Leu Asp Asn Asn Asp
   805 810 815
   Lys Asp Ala Lys Leu Arg Leu Val Val Asp Lys Ser Ser Thr Asp Gly
   820 825 830
   Ile Ala Asp Val Ala Gly Asn Val Ile Lys Glu Lys Asp Ile Leu Ile
   835 840 845 850
   Arg Tyr Asn Ser Trp Arg His Thr Val Ala Ser Val Lys Ala Ala Ala
   855 860 865
   Asp Lys Asp Gly Gln Asn Ala Ser Ala Ala Phe Pro Thr Ser Thr Ala
   870 875 880
   Ile Asn Thr Thr Lys Ser Leu Leu Val Glu Phe Asn Glu Thr Asp Leu
   885 890 895
   Ala Glu Val Lys Pro Glu Asn Ile Val Val Lys Asp Ala Ala Gly Asn
   900 905 910
   Ala Val Ala Gly Thr Val Thr Ala Leu Asp Gly Ser Thr Asn Lys Phe
   915 920 925 930
   Val Phe Thr Pro Ser Gln Glu Leu Lys Ala Gly Thr Val Tyr Ser Val
   935 940 945
   Thr Ile Asp Gly Val Arg Asp Lys Val Gly Asn Thr Ile Ser Lys Tyr
   950 955 960
   Ile Thr Ser Phe Lys Thr Val Ser Ala Asn Pro Thr Leu Ser Ser Ile
   965 970 975
   Ser Ile Ala Asp Gly Ala Val Asn Val Asp Arg Ser Lys Thr Ile Thr
   980 985 990
   Ile Glu Phe Ser Asp Ser Val Pro Asn Pro Thr Ile Thr Leu Lys Lys
   995 1000 1005 1010
   Ala Asp Gly Thr Ser Phe Thr Asn Tyr Thr Leu Val Asn Val Asn Asn
   1015 1020 1025
   Glu Asn Lys Thr Tyr Lys Ile Val Phe His Lys Gly Val Thr Leu Asp
               1030                1035                1040
   Glu Phe Thr Gln Tyr Glu Leu Ala Val Ser Lys Asp Phe Gln Thr Gly
           1045                1050                1055
   Thr Asp Ile Asp Ser Lys Val Thr Phe Ile Thr Gly Ser Val Ala Thr
       1060                1065                1070
   Asp Glu Val Lys Pro Ala Leu Val Gly Val Gly Ser Trp Asn Gly Thr
   1075                1080                1085                1090
   Ser Tyr Thr Gln Asp Ala Ala Ala Thr Arg Leu Arg Ser Val Ala Asp
                   1095                1100                1105
   Phe Val Ala Glu Pro Val Ala Leu Gln Phe Ser Glu Gly Ile Asp Leu
               1110                1115                1120
   Thr Asn Ala Thr Val Thr Val Thr Asn Ile Thr Asp Asp Lys Thr Val
           1125                1130                1135
   Glu Val Ile Ser Lys Glu Ser Val Asp Ala Asp His Asp Ala Gly Ala
       1140                1145                1150
   Thr Lys Glu Thr Leu Val Ile Asn Thr Val Thr Pro Leu Val Leu Asp
   1155                1160                1165                1170
   Asn Ser Lys Thr Tyr Lys Ile Val Val Ser Gly Val Lys Asp Ala Ala
                   1175                1180                1185
   Gly Asn Val Ala Asp Thr Ile Thr Phe Tyr Ile Lys
               1190                1195
(2) ANGABEN ZU SEQ ID NO: 3:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
   TTAATCGATT CTAGATGGAT AGGAAAAAAG CTG                                33
(2) ANGABEN ZU SEQ ID NO: 4:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 4:
   ATACCCGGGG GTACGGATCC GATACAGATT TGAGCAA                            37
(2) ANGABEN ZU SEQ ID NO: 5:
   (i) SEQUENZKENNZEICHEN:
      (A) LÄNGE: 2766 Basenpaare
      (B) ART: Nucleotid
      (C) STRANGFORM: beides
      (D) TOPOLOGIE: linear
   (vi) URSPRÜNLICHE HERKUNFT:
      (A) ORGANISMUS: Bacillus stearothermophilus
      (B) STAMM: PV72
   (vii) UNMITTELBARE HERKUNFT:
      (B) CLON (E) : sbsB
   (ix) MERKMAL:
      (A) NAME/SCHLÜSSEL: CDS
      (B) LAGE:1..2763
   (ix) MERKMAL:
      (A) NAME/SCHLLTSSEL: sig peptide
      (B) LAGE:1..93
   (ix) MERKMAL:
      (A) NAME/SCHLÜSSEL: mat_peptide
      (B) LAGE:94..2763
   (xi) SEQUENZBESCHREIBUNG: SEQ ID NO: 5:
   ATG GCT TAT CAA CCT AAG TCT TTT CGC AAG TTT GTT GCG ACA ACT GCA       48
   Met Ala Tyr Gln Pro Lys Ser Phe Arg Lys Phe Val Ala Thr Thr Ala
   -31 -30                 -25                 -20
   ACA GCT GCC ATT GTA GCA TCT GCG GTA GCT CCT GTA GTA TCT GCA GCA       96
   Thr Ala Ala Ile Val Ala Ser Ala Val Ala Pro Val Val Ser Ala Ala
   -15                 -10                 -5                    1
   AGC TTC ACA GAT GTT GCG CCG CAA TAT AAA GAT GCG ATC GAT TTC TTA      144
   Ser Phe Thr Asp Val Ala Pro Gln Tyr Lys Asp Ala Ile Asp Phe Leu
               5                   10                  15
   GTA TCA ACT GGT GCA ACA AAA GGT AAA ACA GAA ACA AAA TTC GGC GTT      192
   Val Ser Thr Gly Ala Thr Lys Gly Lys Thr Glu Thr Lys Phe Gly Val
           20                  25                  30
   TAC GAT GAA ATC ACT CGT CTA GAT GCG GCA GTT ATT CTT GCA AGA GTA      240
   Tyr Asp Glu Ile Thr Arg Leu Asp Ala Ala Val Ile Leu Ala Arg Val
       35                  40                  45
   TTA AAA CTA GAC GTT GAC AAC GCA AAA GAC GCA GGC TTC ACA GAT GTG      288
   Leu Lys Leu Asp Val Asp Asn Ala Lys Asp Ala Gly Phe Thr Asp Val
    50                 55                  60                  65
   CCA AAA GAC CGT GCA AAA TAC GTC AAC GCG CTT GTA GAA GCT GGC GTA      336
   Pro Lys Asp Arg Ala Lys Tyr Val Asn Ala Leu Val Glu Ala Gly Val
                   70                  75                  80
   TTA AAC GGT AAA GCA CCT GGC AAA TTT GGT GCA TAC GAC CCA TTA ACT      384
   Leu Asn Gly Lys Ala Pro Gly Lys Phe Gly Ala Tyr Asp Pro Leu Thr
               85                  90                  95
   CGC GTT GAA ATG GCA AAA ATC ATC GCG AAC CGT TAC AAA TTA AAA GCT      432
   Arg Val Glu Met Ala Lys Ile Ile Ala Asn Arg Tyr Lys Leu Lys Ala
           100                 105                 110
   GAC GAT GTA AAA CTT CCA TTC ACT GAT GTA AAC GAT ACA TGG GCA CCA      480
   Asp Asp Val Lys Leu Pro Phe Thr Asp Val Asn Asp Thr Trp Ala Pro
       115                 120                 125
   TAC GTA AAA GCG CTT TAT AAA TAC GAA GTA ACC AAA AGG TTA AAA CAC      528
   Tyr Val Lys Ala Leu Tyr Lys Tyr Glu Val Thr Lys Arg Leu Lys His
   130                 135                 140                 145
   CAA CAA GCT TCG GTG CAT ACC AAA AAC ATC ACT CTG CGT GAC TTT GCG      576
   Gln Gln Ala Ser Val His Thr Lys Asn Ile Thr Leu Arg Asp Phe Ala
                   150                 155                 160
   CAA TTT GTA TAT AGA GCG GTG AAT ATT AAT GCA GTG CCA GAA ATA GTT      624
   Gln Phe Val Tyr Arg Ala Val Asn Ile Asn Ala Val Pro Glu Ile Val
               165                 170                 175
   GAA GTA ACT GCG GTT AAT TCG ACT ACA GTG AAA GTA ACA TTC AAT ACG      672
   Glu Val Thr Ala Val Asn Ser Thr Thr Val Lys Val Thr Phe Asn Thr
           180                 185                 190
   CAA ATT GCT GAT GTT GAT TTC ACA AAT TTT GCT ATC GAT AAC GGT TTA      720
   Gln Ile Ala Asp Val Asp Phe Thr Asn Phe Ala Ile Asp Asn Gly Leu
       195                 200                 205
   ACT GTT ACT AAA GCA ACT CTT TCT CGT GAT AAA AAA TCC GTA GAG GTT      768
   Thr Val Thr Lys Ala Thr Leu Ser Arg Asp Lys Lys Ser Val Glu Val
   210                 215                 220                 225
   GTG GTA AAT AAA CCG TTT ACT CGT AAT CAG GAA TAT ACA ATT ACA GCG      816
   Val Val Asn Lys Pro Phe Thr Arg Asn Gln Glu Tyr Thr Ile Thr Ala
                   230                 235                 240
   ACA GGC ATT AAA AAT TTA AAA GGC GAG ACC GCT AAG GAA TTA ACT GGT      864
   Thr Gly Ile Lys Asn Leu Lys Gly Glu Thr Ala Lys Glu Leu Thr Gly
               245                 250                 255
   AAG TTT GTT TGG TCT GTT CAA GAT GCG GTA ACT GTT GCA CTA AAT AAT      912
   Lys Phe Val Trp Ser Val Gln Asp Ala Val Thr Val Ala Leu Asn Asn
           260                 265                 270
   AGT TCG CTT AAA GTT GGA GAG GAA TCT GGT TTA ACT GTA AAA GAT CAG      960
   Ser Ser Leu Lys Val Gly Glu Glu Ser Gly Leu Thr Val Lys Asp Gln
       275                 280                 285
   GAT GGC AAA GAT GTT GTA GGT GCT AAA GTA GAA CTT ACT TCT TCT AAT     1008
   Asp Gly Lys Asp Val Val Gly Ala Lys Val Glu Leu Thr Ser Ser Asn
   290                 295                 300                 305
   ACT AAT ATT GTT GTA GTT TCA AGT GGC GAA GTA TCA GTA TCT GCT GCT     1056
   Thr Asn Ile Val Val Val Ser Ser Gly Glu Val Ser Val Ser Ala Ala
                   310                 315                 320
   AAA GTT ACA GCT GTA AAA CCG GGA ACA GCT GAT GTT ACT GCA AAA GTT 1104
   Lys Val Thr Ala Val Lys Pro Gly Thr Ala Asp Val Thr Ala Lys Val
   325 330 335
   ACA TTA CCA GAT GGT GTT GTA CTA ACA AAT ACA TTT AAA GTG ACA GTT 1152
   Thr Leu Pro Asp Gly Val Val Leu Thr Asn Thr Phe Lys Val Thr Val
   340 345 350
   ACA GAA GTG CCT GTT CAA GTC CAA AAT CAA GGA TTT ACT TTA GTT GAT 1200
   Thr Glu Val Pro Val Gln Val Gln Asn Gln Gly Phe Thr Leu Val Asp
   355 360 365
   AAT CTT TCT AAT GCT CCA CAG AAT ACA GTT GCA TTT AAC AAA GCT GAG 1248
   Asn Leu Ser Asn Ala Pro Gln Asn Thr Val Ala Phe Asn Lys Ala Glu
   370 375 380 385
   AAA GTA ACT TCA ATG TTT GCT GGA GAA ACT AAA ACA GTT GCA ATG TAT 1296
   Lys Val Thr Ser Met Phe Ala Gly Glu Thr Lys Thr Val Ala Met Tyr
   390 395 400
   GAT ACT AAA AAC GGT GAT CCT GAA ACT AAA CCT GTT GAT TTC AAA GAT 1344
   Asp Thr Lys Asn Gly Asp Pro Glu Thr Lys Pro Val Asp Phe Lys Asp
   405 410 415
   GCA ACT GTA CGT TCA TTA AAT CCA ATT ATT GCA ACA GCT GCT ATT AAT 1392
   Ala Thr Val Arg Ser Leu Asn Pro Ile Ile Ala Thr Ala Ala Ile Asn
   420 425 430
   GGT AGT GAG CTC CTT GTC ACA GCT AAT GCT GGC CAA TCT GGA AAA GCT 1440
   Gly Ser Glu Leu Leu Val Thr Ala Asn Ala Gly Gln Ser Gly Lys Ala
   435 440 445
   TCA TTT GAA GTA ACA TTA AAA GAT AAT ACA AAA AGA ACA TTT ACA GTT 1488
   Ser Phe Glu Val Thr Leu Lys Asp Asn Thr Lys Arg Thr Phe Thr Val
   450 455 460 465
   GAT GTA AAA AAA GAC CCT GTA TTA CAA GAT ATA AAA GTA GAT GCA ACT 1536
   Asp Val Lys Lys Asp Pro Val Leu Gln Asp Ile Lys Val Asp Ala Thr
   470 475 480
   TCT GTT AAA CTT TCC GAT GAA GCT GTT GGC GGC GGG GAA GTT GAA GGA 1584
   Ser Val Lys Leu Ser Asp Glu Ala Val Gly Gly Gly Glu Val Glu Gly
   485 490 495
   GTT AAC CAA AAA ACG ATT AAA GTA AGT GCA GTT GAC CAA TAC GGT AAA 1632
   Val Asn Gln Lys Thr Ile Lys Val Ser Ala Val Asp Gln Tyr Gly Lys
   500 505 510
   GAA ATT AAA TTT GGT ACA AAA GGT AAA GTT ACT GTT ACA ACT AAT ACA 1680
   Glu Ile Lys Phe Gly Thr Lys Gly Lys Val Thr Val Thr Thr Asn Thr
   515 520 525
   GAA GGA CTA GTT ATT AAA AAT GTA AAT AGC GAT AAT ACA ATT GAC TTT 1728
   Glu Gly Leu Val Ile Lys Asn Val Asn Ser Asp Asn Thr Ile Asp Phe
   530 535 540 545
   GAT AGC GGC AAT AGT GCA ACT GAC CAA TTT GTT GTC GTT GCA ACA AAA 1776
   Asp Ser Gly Asn Ser Ala Thr Asp Gln Phe Val Val Val Ala Thr Lys
   550 555 560
   GAC AAA ATT GTC AAT GGT AAA GTA GAA GTT AAA TAT TTC AAA AAT GCT 1824
   Asp Lys Ile Val Asn Gly Lys Val Glu Val Lys Tyr Phe Lys Asn Ala
   565 570 575
   AGT GAC ACA ACA CCA ACT TCA ACT AAA ACA ATT ACT GTT AAT GTA GTA 1872
   Ser Asp Thr Thr Pro Thr Ser Thr Lys Thr Ile Thr Val Asn Val Val
   580 585 590
   AAT GTA AAA GCT GAC GCT ACA CCA GTA GGA TTA GAT ATT GTA GCA CCT     1920
   Asn Val Lys Ala Asp Ala Thr Pro Val Gly Leu Asp Ile Val Ala Pro
       595                 600                 605
   TCT AAA ATT GAT GTA AAT GCT CCA AAC ACT GCT TCT ACT GCA GAT GTT     1968
   Ser Lys Ile Asp Val Asn Ala Pro Asn Thr Ala Ser Thr Ala Asp Val
   610                 615                 620                 625
   GAT TTT ATA AAT TTC GAA AGT GTT GAG ATT TAC ACA CTC GAT TCA AAT     2016
   Asp Phe Ile Asn Phe Glu Ser Val Glu Ile Tyr Thr Leu Asp Ser Asn
                   630                 635                 640
   GGT AGA CGT CAA AAA AAA GTT ACT CCA ACT GCA ACT ACA CTT GTA GGT     2064
   Gly Arg Arg Gln Lys Lys Val Thr Pro Thr Ala Thr Thr Leu Val Gly
               645                 650                 655
   ACA AAA AAA AAA AAA AAA GTT AAT GGG AAT GTA TTA CAA TTC AAG GGG     2112
   Thr Lys Lys Lys Lys Lys Val Asn Gly Asn Val Leu Gln Phe Lys Gly
           660                 665                 670
   AAC GAA GAA TTA ACG CTA TCA ACT TCT TCT AGT ACA GGA AAC GTA GAT     2160
   Asn Glu Glu Leu Thr Leu Ser Thr Ser Ser Ser Thr Gly Asn Val Asp
       675                 680                 685
   GGA ACA GCA GAA GGA ATG ACA AAA CGT ATT CCA GGG AAA TAT ATC AAC     2208
   Gly Thr Ala Glu Gly Met Thr Lys Arg Ile Pro Gly Lys Tyr Ile Asn
   690                 695                 700                 705
   TCT GCA AGT GTA CCT GCC AGT GCA ACA GTA GCA ACA AGT CCT GTT ACT     2256
   Ser Ala Ser Val Pro Ala Ser Ala Thr Val Ala Thr Ser Pro Val Thr
                   710                 715                 720
   GTA AAG CTT AAT TCA AGT GAT AAT GAT TTA ACA TTT GAA GAA TTA ATA     2304
   Val Lys Leu Asn Ser Ser Asp Asn Asp Leu Thr Phe Glu Glu Leu Ile
               725                 730                 735
   TTC GGT GTA ATT GAC CCT ACA CAA TTA GTC AAA GAT GAA GAC ATC AAC     2352
   Phe Gly Val Ile Asp Pro Thr Gln Leu Val Lys Asp Glu Asp Ile Asn
           740                 745                 750
   GAA TTT ATT GCA GTT TCA AAA GCG GCT AAA AAT GAT GGA TAT TTG TAT     2400
   Glu Phe Ile Ala Val Ser Lys Ala Ala Lys Asn Asp Gly Tyr Leu Tyr
       755                 760                 765
   AAT AAA CCG CTT GTA ACG GTT AAA GAT GCA TCA GGA AAA GTT ATT CCA     2448
   Asn Lys Pro Leu Val Thr Val Lys Asp Ala Ser Gly Lys Val Ile Pro
   770                 775                 780                 785
   ACA GGT GCA AAT GTT TAC GGT CTA AAT CAT GAT GCA ACT AAC GGA AAC     2496
   Thr Gly Ala Asn Val Tyr Gly Leu Asn His Asp Ala Thr Asn Gly Asn
                   790                 795                 800
   ATT TGG TTT GAT GAG GAA CAA GCT GGC TTA GCT AAA AAA TTT AGT GAT     2544
   Ile Trp Phe Asp Glu Glu Gln Ala Gly Leu Ala Lys Lys Phe Ser Asp
               805                 810                 815
   GTA CAT TTT GAT GTT GAT TTT TCA TTA ACT AAC GTT GTA AAA ACT GGT     2592
   Val His Phe Asp Val Asp Phe Ser Leu Thr Asn Val Val Lys Thr Gly
           820                 825                 830
   AGC GGT ACA GTT TCT TCA TCG CCA TCA TTA TCT GAC GCA ATT CAA CTT     2640
   Ser Gly Thr Val Ser Ser Ser Pro Ser Leu Ser Asp Ala Ile Gln Leu
       835                 840                 845
   ACT AAT TCA GGC GAT GCA GTA TCG TTT ACA TTA GTT ATC AAA TCA ATT     2688
   Thr Asn Ser Gly Asp Ala Val Ser Phe Thr Leu Val Ile Lys Ser Ile
   850                 855                 860                 865
   TAT GTT AAA GGC GCA GAT AAA GAT GAT AAT AAC TTA CTT GCA GCC CCT        2736
   Tyr Val Lys Gly Ala Asp Lys Asp Asp Asn Asn Leu Leu Ala Ala Pro
                   870                 875                 880
   GTT TCT GTC AAT GTG ACT GTG ACA AAA TAA                                2766
   Val Ser Val Asn Val Thr Val Thr Lys
               885                 890
(2) ANGABEN ZU SEQ ID NO: 6:
   (i) SEQUENZKENNZEICHEN:
      (A) LÄNGE: 921 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 6:
   Met Ala Tyr Gln Pro Lys Ser Phe Arg Lys Phe Val Ala Thr Thr Ala
   -31 -30                 -25                 -20
   Thr Ala Ala Ile Val Ala Ser Ala Val Ala Pro Val Val Ser Ala Ala
   -15                 -10                  -5                   1
   Ser Phe Thr Asp Val Ala Pro Gln Tyr Lys Asp Ala Ile Asp Phe Leu
                 5                  10                  15
   Val Ser Thr Gly Ala Thr Lys Gly Lys Thr Glu Thr Lys Phe Gly Val
            20                  25                  30
   Tyr Asp Glu Ile Thr Arg Leu Asp Ala Ala Val Ile Leu Ala Arg Val
        35                  40                  45
   Leu Lys Leu Asp Val Asp Asn Ala Lys Asp Ala Gly Phe Thr Asp Val
    50                  55                  60                  65
   Pro Lys Asp Arg Ala Lys Tyr Val Asn Ala Leu Val Glu Ala Gly Val
                    70                  75                  80
   Leu Asn Gly Lys Ala Pro Gly Lys Phe Gly Ala Tyr Asp Pro Leu Thr
                85                  90                  95
   Arg Val Glu Met Ala Lys Ile Ile Ala Asn Arg Tyr Lys Leu Lys Ala
           100                 105                 110
   Asp Asp Val Lys Leu Pro Phe Thr Asp Val Asn Asp Thr Trp Ala Pro
       115                 120                 125
   Tyr Val Lys Ala Leu Tyr Lys Tyr Glu Val Thr Lys Arg Leu Lys His
   130                 135                 140                 145
   Gln Gln Ala Ser Val His Thr Lys Asn Ile Thr Leu Arg Asp Phe Ala
                   150                 155                 160
   Gln Phe Val Tyr Arg Ala Val Asn Ile Asn Ala Val Pro Glu Ile Val
               165                 170                 175
   Glu Val Thr Ala Val Asn Ser Thr Thr Val Lys Val Thr Phe Asn Thr
           180                 185                 190
   Gln Ile Ala Asp Val Asp Phe Thr Asn Phe Ala Ile Asp Asn Gly Leu
       195                 200                 205
   Thr Val Thr Lys Ala Thr Leu Ser Arg Asp Lys Lys Ser Val Glu Val
   210                 215                 220                 225
   Val Val Asn Lys Pro Phe Thr Arg Asn Gln Glu Tyr Thr Ile Thr Ala
                   230                 235                 240
   Thr Gly Ile Lys Asn Leu Lys Gly Glu Thr Ala Lys Glu Leu Thr Gly
               245                 250                 255
   Lys Phe Val Trp Ser Val Gln Asp Ala Val Thr Val Ala Leu Asn Asn
           260                 265                 270
   Ser Ser Leu Lys Val Gly Glu Glu Ser Gly Leu Thr Val Lys Asp Gln
       275                 280                 285
   Asp Gly Lys Asp Val Val Gly Ala Lys Val Glu Leu Thr Ser Ser Asn
   290                 295                 300                 305
   Thr Asn Ile Val Val Val Ser Ser Gly Glu Val Ser Val Ser Ala Ala
                   310                 315                 320
   Lys Val Thr Ala Val Lys Pro Gly Thr Ala Asp Val Thr Ala Lys Val
               325                 330                 335
   Thr Leu Pro Asp Gly Val Val Leu Thr Asn Thr Phe Lys Val Thr Val
           340                 345                 350
   Thr Glu Val Pro Val Gln Val Gln Asn Gln Gly Phe Thr Leu Val Asp
       355                 360                 365
   Asn Leu Ser Asn Ala Pro Gln Asn Thr Val Ala Phe Asn Lys Ala Glu
   370                 375                 380                 385
   Lys Val Thr Ser Met Phe Ala Gly Glu Thr Lys Thr Val Ala Met Tyr
                   390                 395                 400
   Asp Thr Lys Asn Gly Asp Pro Glu Thr Lys Pro Val Asp Phe Lys Asp
               405                 410                 415
   Ala Thr Val Arg Ser Leu Asn Pro Ile Ile Ala Thr Ala Ala Ile Asn
           420                 425                 430
   Gly Ser Glu Leu Leu Val Thr Ala Asn Ala Gly Gln Ser Gly Lys Ala
       435                 440                 445
   Ser Phe Glu Val Thr Leu Lys Asp Asn Thr Lys Arg Thr Phe Thr Val
   450                 455                 460                 465
   Asp Val Lys Lys Asp Pro Val Leu Gln Asp Ile Lys Val Asp Ala Thr
                   470                 475                 480
   Ser Val Lys Leu Ser Asp Glu Ala Val Gly Gly Gly Glu Val Glu Gly
               485                 490                 495
   Val Asn Gln Lys Thr Ile Lys Val Ser Ala Val Asp Gln Tyr Gly Lys
           500                 505                 510
   Glu Ile Lys Phe Gly Thr Lys Gly Lys Val Thr Val Thr Thr Asn Thr
       515                 520                 525
   Glu Gly Leu Val Ile Lys Asn Val Asn Ser Asp Asn Thr Ile Asp Phe
   530                 535                 540                 545
   Asp Ser Gly Asn Ser Ala Thr Asp Gln Phe Val Val Val Ala Thr Lys
   550 555 560
   Asp Lys Ile Val Asn Gly Lys Val Glu Val Lys Tyr Phe Lys Asn Ala
   565 570 575
   Ser Asp Thr Thr Pro Thr Ser Thr Lys Thr Ile Thr Val Asn Val Val
   580 585 590
   Asn Val Lys Ala Asp Ala Thr Pro Val Gly Leu Asp Ile Val Ala Pro
   595 600 605
   Ser Lys Ile Asp Val Asn Ala Pro Asn Thr Ala Ser Thr Ala Asp Val
   610 615 620 625
   Asp Phe Ile Asn Phe Glu Ser Val Glu Ile Tyr Thr Leu Asp Ser Asn
   630 635 640
   Gly Arg Arg Gln Lys Lys Val Thr Pro Thr Ala Thr Thr Leu Val Gly
   645 650 655
   Thr Lys Lys Lys Lys Lys Val Asn Gly Asn Val Leu Gln Phe Lys Gly
   660 665 670
   Asn Glu Glu Leu Thr Leu Ser Thr Ser Ser Ser Thr Gly Asn Val Asp
   675 680 685
   Gly Thr Ala Glu Gly Met Thr Lys Arg Ile Pro Gly Lys Tyr Ile Asn
   690 695 700 705
   Ser Ala Ser Val Pro Ala Ser Ala Thr Val Ala Thr Ser Pro Val Thr
   710 715 720
   Val Lys Leu Asn Ser Ser Asp Asn Asp Leu Thr Phe Glu Glu Leu Ile
   725 730 735
   Phe Gly Val Ile Asp Pro Thr Gln Leu Val Lys Asp Glu Asp Ile Asn
   740 745 750
   Glu Phe Ile Ala Val Ser Lys Ala Ala Lys Asn Asp Gly Tyr Leu Tyr
   755 760 765
   Asn Lys Pro Leu Val Thr Val Lys Asp Ala Ser Gly Lys Val Ile Pro
   770 775 780 785
   Thr Gly Ala Asn Val Tyr Gly Leu Asn His Asp Ala Thr Asn Gly Asn
   790 795 800
   Ile Trp Phe Asp Glu Glu Gln Ala Gly Leu Ala Lys Lys Phe Ser Asp
   805 810 815
   Val His Phe Asp Val Asp Phe Ser Leu Thr Asn Val Val Lys Thr Gly
   820 825 830
   Ser Gly Thr Val Ser Ser Ser Pro Ser Leu Ser Asp Ala Ile Gln Leu
   835 840 845
   Thr Asn Ser Gly Asp Ala Val Ser Phe Thr Leu Val Ile Lys Ser Ile
   850 855 860 865
   Tyr Val Lys Gly Ala Asp Lys Asp Asp Asn Asn Leu Leu Ala Ala Pro
   870 875 880
   Val Ser Val Asn Val Thr Val Thr Lys
   885 890
(2) ANGABEN ZU SEQ ID NO: 7:
   (i) SEQUENZKENNZEICHEN:
      (A) LÄNGE: 498 Basenpaare
      (B) ART: Nucleotid
      (C) STRANGFORM: beides
      (D) TOPOLOGIE: linear
   (xi) SEQUENZBESCHREIBUNG: SEQ ID NO: 7:
   CCCATGGACC CGTCCAAGGA CTCCAAAGCT CAGGTTTCTG CAGCCGAAGC TGGTATCACT    60
   GGCACCTGGT ATAACCAACT GGGGTCGACT TTCATTGTGA CCGCTGGTGC GGACGGAGCT   120
   CTGACTGGCA CCTACGAATC TGCGGTTGGT AACGCAGAAT CCCGCTACGT ACTGACTGGC   180
   CGTTATGACT CTGCACCTGC CACCGATGGC TCTGGTACCG CTCTGGGCTG GACTGTGGCT   240
   TGGAAAAACA ACTATCGTAA TGCGCACAGC GCCACTACGT GGTCTGGCCA ATACGTTGGC   300
   GGTGCTGAGG CTCGTATCAA CACTCAGTGG CTGTTAACAT CCGGCACTAC CGAAGCGAAT   360
   GCATGGAAAT CGACACTAGT AGGTCATGAC ACCTTTACCA AAGTTAAGCC TTCTGCTGCT   420
   AGCATTGATG CTGCCAAGAA AGCAGGCGTA AACAACGGTA ACCCTCTAGA CGCTGTTCAG   480
   CAATAATAAG GATCCGGG                                                 498
(2) ANGABEN ZU SEQ ID NO: 8:
   (i) SEQUENZKENNZEICHEN:
      (A) LÄNGE: 29 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 8:
   TTCATCGTAA ACGCCGAATT TTGTTTCTG    29
(2) ANGABEN ZU SEQ ID NO: 9:
   (i) SEQUENZKENNZEICHEN:
      (A) LÄNGE: 26 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 9:
   AGGGAAATAT ATCAACTCTG CAAGTG    26